# EUROPEAN PATENT SPECIFICATION

(11) **EP 1 421 070 B1**
(45) Date of publication and mention of the grant of the patent: **26.11.2008**
(21) Application number: 02760961.9
(22) Date of filing: 21.08.2002
(51) Int. Cl.: C07D 265/30, C07D 211/20, C07D 401/12, C07D 403/12, C07D 413/12, C07D 417/12, A61K 31/4523, A61K 31/5377, A61P 11/00, A61P 19/00, A61P 31/00, A61P 37/00

(54) **NEW PIPERIDINYL DERIVATES AS MODULATORS OF CHEMOKINE RECEPTOR ACTIVITY**
NEUE PIPERIDINYLDERIVATE ALS MODULATOREN DER AKTIVITÄT DES CHEMOKINREZEPTORS
NOUVEAUX DERIVES DE PIPERIDINYLE, UTILISES EN TANT QUE MODULATEURS DE L'ACTIVITE D'UN RECEPTEUR DE CHIMIOKINE

(30) Priority: 22.08.2001 SE 0102809
(43) Date of publication of application: 26.05.2004
(60) Divisional application: 08165498.0
(73) Proprietor: AstraZeneca AB, 151 85 Södertälje (SE)
(72) Inventor: GUSTAFSSON, Jörgen, S-221 87 Lund (SE); HOSSAIN, Nafizal, S-221 87 Lund (SE); NILSSON, Stinabritt, S-221 87 Lund (SE)
(86) International application number: PCT/SE2002/001488
(87) International publication number: WO 2003/018566

(56) References cited:
- WO-A1-00/58305
- WO-A1-01/14333

## Description

The present invention relates to novel compounds, processes for their preparation, pharmaceutical compositions containing them and their use in therapy.

Chemokines play an important role in immune and inflammatory responses in various diseases and disorders, including asthma and allergic diseases, as well as autoimmune pathologies such as rheumatoid arthritis and atherosclerosis. These small secreted molecules are a growing superfamily of 8-14 kDa proteins characterised by a conserved four cysteine motif. The chemokine superfamily can be divided into two main groups exhibiting characteristic structural motifs, the Cys-X-Cys (C-X-C) and Cys-Cys (C-C) families. These are distinguished on the basis of a single amino acid insertion between the NH-proximal pair of cysteine residues and sequence similarity.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils such as human monocyte chemotactic proteins 1-3 (MCP-1, MCP-2 and MCP-3), RANTES (Regulated on Activation, Normal T Expressed and Secreted), eotaxin and the macrophage inflammatory proteins 1α and 1β (MIP-1α and MIP-1β).

Studies have demonstrated that the actions of the chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10, CXCR1, CXCR2, CXCR3 and CXCR4. These receptors represent good targets for drug development since agents which modulate these receptors would be useful in the treatment of disorders and diseases such as those previously mentioned.

Certain cyclohexylphenoxymorpholines are known from CAPLUS accession no. 1980:586265, Document No. 93:186265 (Farmaco, Ed. Sci. (1980), 35(6), 504-26) which are said to have antidepressant, tranquilizer, analgesic and spasmolytic activity.

In accordance with the present invention, there is provided a compound of formula 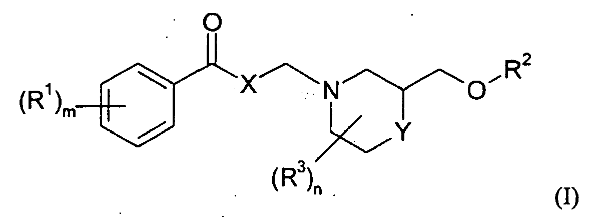 wherein
m is 0, 1, 2 or 3;
each R¹ independently represents halogen, cyano, nitro, carboxyl, hydroxyl, C₃-C₆ cycloalkyl, C₁-C₆ alkoxy, C₁-C₆ alkoxycarbonyl, C₁-C₆ haloalkyl, C₁-C₆ haloalkoxy, -NR⁶R⁷, C₃-C₆ cycloalkylamino, C₁-C₆ alkylthio, C₁-C₆ alkylcarbonyl, C₁-C₆ alkylcarbonylamino, sulphonamido (-SO₂NH₂), C₁-C₆ alkylsulphonyl, -C(O)NR⁸R⁹, -NR¹⁰C(O)-(NH)ₚR¹¹, phenyl, or C₁-C₆ alkyl optionally substituted by at least one substituent selected from carboxyl and C₁-C₆ alkoxycarbonyl;
X represents a bond or a group CH₂ or (CH₂)₂;
Y represents an oxygen atom or a group CH₂;
R² represents an unsaturated 5- to 10-membered ring system which may comprise at least one ring heteroatom selected from nitrogen, oxygen and sulphur, the ring system being optionally substituted by at least one substituent selected from halogen, cyano, nitro, carboxyl, hydroxyl, C₂-C₆ alkenyl, C₃-C₅ cycloalkyl, C₁-C₆ alkoxy, C₁-C₆ alkoxycarbonyl, C₁-C₆ haloalkyl, C₁-C₆ haloalkoxy, -NR¹²R¹³, C₃-C₆ cycloalkylamino, C₁-C₆ alkylthio, C₁-C₆ alkylcarbonyl, phenylcarbonyl, C₁-C₆ alkylcarbonylamino, sulphonamido (-SO₂NH₂), C₁-C₆ alkylsulphonyl, -C(O)NR¹⁴R¹⁵, C₁-C₆ alkoxycarbonylC₁-C₆ alkyl, phenyl, isoxazolyl, pyrazolyl, -NHSO₂CH₃, -NHC(O)NR¹⁶R¹⁷, -OC(O)NR¹⁸R¹⁹, -OCH₂C(O)NR²⁰R²¹, -NHC(O)OR²², NHC(O)R²³, and C₁-C₆ alkyl itself optionally substituted by at least one substituent selected from carboxyl and C₁-C₆ alkoxycarbonyl;
n is 0, 1 or 2;
each R³ independently represents a C₁-C₆ alkyl, C₁-C₆ alkoxycarbonyl, -CH₂OH or carboxyl group;
R⁶ and R⁷ each independently represent a hydrogen atom or a C₁-C₆ alkyl group, or R⁶ and R⁷ together with the nitrogen atom to which they are attached form a 4- to 7-membered saturated heterocycle;
R⁸ and R⁹ each independently represent a hydrogen atom or a C₁-C₆ alkyl group optionally substituted by at least one C₁-C₆ alkoxycarbonyl;
p is 0 or 1;
R¹⁰ represents a hydrogen atom or a C₁-C₆ alkyl group;
R¹¹ represents a hydrogen atom, or a C₁-C₆ alkyl group optionally substituted by at least one substituent selected from carboxyl, C₁-C₆ alkoxy and C₁-C₆ alkoxycarbonyl;
R¹² and R¹³ each independently represent a hydrogen atom, or a C₁-C₆ alkyl group optionally substituted by at least one substituent selected from phenyl, carboxyl and C₁-C₆ alkoxycarbonyl, or R¹² and R¹³ together with the nitrogen atom to which they are attached form a 4- to 7-membered saturated heterocycle;
R¹⁴ and R¹⁵ each independently represent a hydrogen atom, a C₃-C₆ cycloalkyl group, or a C₁-C₆ alkyl group optionally substituted by at least one substituent selected from carboxyl and C₁-C₆ alkoxycarbonyl, or R¹⁴ and R¹⁵ together with the nitrogen atom to which they are attached form a 4- to 7-membered saturated heterocycle;
R¹⁶ and R¹⁷ each independently represent a hydrogen atom, or a C₁-C₆ alkyl group optionally substituted by at least one substituent selected from carboxyl and C₁-C₆ alkoxycarbonyl, or R¹⁶ and R¹⁷ together with the nitrogen atom to which they are attached form a 4- to 7-membered saturated heterocycle;
R¹⁸ and R¹⁹ each independently represent a hydrogen atom, or a C₁-C₆ alkyl group optionally substituted by at least one substituent selected from carboxyl and C₁-C₆ alkoxycarbonyl, or R¹⁸ and R¹⁹ together with the nitrogen atom to which they are attached form a 4- to 7-membered saturated heterocycle;
R²⁰ and R²¹ each independently represent a hydrogen atom, or a C₁-C₆ alkyl group optionally substituted by at least one substituent selected from carboxyl and C₁-C₆ alkoxycarbonyl, or R²⁰ and R²¹ together with the nitrogen atom to which they are attached form a 4- to 7-membered saturated heterocycle;
R²² represents a hydrogen atom, or a C₁-C₆ alkyl group optionally substituted by at least one substituent selected from carboxyl and C₁-C₆ alkoxycarbonyl;
R²³ represents a group C₁-C₆ alkyl, C₂-C₆ alkenyl, C₃-C₆ cycloalkyl, adamantyl, C₅-C₆ cycloalkenyl, phenyl or a saturated or unsaturated 5- to 10-membered heterocyclic ring system comprising at least one ring heteroatom selected from nitrogen, oxygen and sulphur, each group being optionally substituted by at least one substituent selected from nitro, hydroxyl, oxo, halogen, carboxyl, C₁-C₆ alkyl, C₁-C₆ alkoxy, C₁-C₆ alkylthio, C₁-C₆ alkylcarbonyl, C₁-C₆ alkoxycarbonyl, phenyl and -NHC(O)-R²⁴; and
R²⁴ represents a C₁-C₆ alkyl, amino (-NH₂) or phenyl group;
or a pharmaceutically acceptable salt or solvate thereof.

In the context of the present specification, an alkyl substituent group or an alkyl moiety in a substituent group may be linear or branched. A haloalkyl or haloalkoxy substituent group will comprise at least one halogen atom, e.g. one, two, three or four halogen atoms. When R⁶ and R⁷, or R¹² and R¹³, or R¹⁴ and R¹⁵, or R¹⁶ and R¹⁷, or R¹⁸ and R¹⁹, or R²⁰ and R²¹ represent a saturated heterocycle, it should be understood that the only heteroatom present is the nitrogen atom to which R⁶ and R⁷, or R¹² and R¹³, or R¹⁴ and R¹⁵, or R¹⁶ and R¹⁷, or R¹⁸ and R¹⁹, or R²⁰ and R²¹ are attached. In the definition of R²³, it should be noted that the saturated or unsaturated 5- to 10-membered heterocyclic ring system may have alicyclic or aromatic properties.

In one embodiment, the integer m is 1 or 2.

Each R¹ independently represents halogen (e.g. chlorine, fluorine, bromine or iodine), cyano, nitro, carboxyl, hydroxyl, C₃-C₆ cycloalkyl (cyclopropyl, cyclobutyl, cyclopentyl or cyclohexyl), C₁-C₆, preferably C₁-C₄, alkoxy (e.g. methoxy, ethoxy, n-propoxy or n-butoxy), C₁-C₆, preferably C₁-C₄, alkoxycarbonyl (e.g. methoxycarbonyl or ethoxycarbonyl), C₁-C₆, preferably C₁-C₄, haloalkyl (e.g. trifluoromethyl), C₁-C₆, preferably C₁-C₄, haloalkoxy (e.g. trifluoromethoxy), -NR⁶R⁷, C₃-C₆ cycloalkylamino (e.g. cyclopropylamino, cyclobutylamino, cyclopentylamino or cyclohexylamino), C₁-C₆, preferably C₁-C₄, alkylthio (e.g. methylthio or ethylthio), C₁-C₆, preferably C₁-C₄, alkylcarbonyl (e.g. methylcarbonyl, ethylcarbonyl, n-propylcarbonyl, isopropylcarbonyl, n-butylcarbonyl, n-pentylcarbonyl or n-hexylcarbonyl), C₁-C₆, preferably C₁-C₄, alkylcarbonylamino (e.g. methylcarbonylamino or ethylcarbonylamino), sulphonamido, C₁-C₆, preferably C₁-C₄, alkylsulphonyl (e.g. methylsulphonyl, ethylsulphonyl, n-propylsulphonyl, isopropylsulphonyl, n-butylsulphonyl, n-pentylsulphonyl or n-hexylsulphonyl), -C(O)NR⁸R⁹, -NR¹⁰C(O)-(NH)ₚR¹¹, phenyl, or C₁-C₆, preferably C₁-C₄, alkyl (e.g. methyl, ethyl, n-propyl, isopropyl, n-butyl, isobutyl, tert-butyl, n-pentyl or n-hexyl) optionally substituted by at least one substituent (e.g. one or two substituents independently) selected from carboxyl and C₁-C₆, preferably C₁-C₄, alkoxycarbonyl (e.g. methoxycarbonyl or ethoxycarbonyl).

In an embodiment of the invention, each R¹ independently represents halogen (particularly chlorine or fluorine), cyano, nitro, C₁-C₆ alkoxy (particularly methoxy), C₁-C₆ alkylcarbonyl (particularly methylcarbonyl) or C₁-C₆ alkylcarbonylamino (particularly methylcarbonylamino). In another embodiment, each R¹ represents a halogen atom.

R² represents an unsaturated 5- to 10-membered ring system which may comprise at least one ring heteroatom (e.g. one, two or three ring heteroatoms independently) selected from nitrogen, oxygen and sulphur, the ring system being optionally substituted by at least one substituent (e.g. one, two, three or four substituents independently) selected from halogen (e.g. chlorine, fluorine, bromine or iodine), cyano, nitro, carboxyl, hydroxyl, C₂-C₆ alkenyl (e.g. ethenyl or 2-propenyl), C₃-C₅ cycloalkyl (cyclopropyl, cyclobutyl or cyclopentyl), C₁-C₆, preferably C₁-C₄, alkoxy (e.g. methoxy, ethoxy, n-propoxy or n-butoxy), C₁-C₆, preferably C₁-C₄, alkoxycarbonyl (e.g. methoxycarbonyl or ethoxycarbonyl), C₁-C₆, preferably C₁-C₄, haloalkyl (e.g. trifluoromethyl), C₁-C₆, preferably C₁-C₄, haloalkoxy (e.g. trifluoromethoxy), -NR¹²R¹³, C₃-C₆ cycloalkylamino (e.g. cyclopropylamino, cyclobutylamino, cyclopentylamino or cyclohexylamino), C₁-C₆, preferably C₁-C₄, alkylthio (e.g. methylthio or ethylthio), C₁-C₆, preferably C₁-C₄, alkylcarbonyl (e.g. methylcarbonyl, ethylcarbonyl, n-propylcarbonyl, isopropylcarbonyl, n-butylcarbonyl, n-pentylcarbonyl or n-hexylcarbonyl), phenylcarbonyl, C₁-C₆, preferably C₁-C₄, alkylcarbonylamino (e.g. methylcarbonylamino or ethylcarbonylamino), sulphonamido, C₁-C₆, preferably C₁-C₄, alkylsulphonyl (e.g. methylsulphonyl, ethylsulphonyl, n-propylsulphonyl, isopropylsulphonyl, n-butylsulphonyl, n-pentylsulphonyl or n-hexylsulphonyl), -C(O)NR¹⁴R¹⁵, C₁-C₆ alkoxycarbonylC₁-C₆ alkyl, preferably C₁-C₄ alkoxycarbonylC₁-C₄ alkyl (e.g. methoxycarbonylmethyl or methoxycarbonylethyl), phenyl, isoxazolyl, pyrazolyl, -NHSO₂CH₃, -NHC(O)NR¹⁶R¹⁷, -OC(O)NR¹⁸R¹⁹, -OCH₂C(O)NR²⁰R²¹, -NHC(O)OR²², -NHC(O)R²³, and C₁-C₆, preferably C₁-C₄, alkyl (e.g. methyl, ethyl, n-propyl, isopropyl, n-butyl, isobutyl, tert-butyl, n-pentyl or n-hexyl) itself optionally substituted by at least one (e.g. one or two substituents independently) selected from carboxyl and C₁-C₆, preferably C₁-C₄, alkoxycarbonyl (e.g. methoxycarbonyl or ethoxycarbonyl).

The unsaturated 5- to 10-membered ring system in R² may be monocyclic or polycyclic (fused or otherwise), e.g. bicyclic, examples of which include phenyl, naphthyl, 1,3-benzodioxolyl, pyrazolyl, thienyl, oxazolyl, imidazolyl, pyridinyl, pyridopyrrolyl, benzimidazolyl, indazolyl, benzothiazolyl, quinolinyl, isoquinolinyl, quinazolinyl, quinoxalinyl, benzisoxazolyl, benzoxazolyl, thiazolyl and benzotriazolyl. In one embodiment, the ring system is selected from the group consisting of phenyl, benzothiazolyl, quinolinyl, quinazolinyl, naphthyl, benzisoxazolyl and benzoxazolyl. Alternatively, the ring system in R² is monocyclic and 5- or 6-membered, especially phenyl.

In one embodiment, the unsaturated 5- to 10-membered ring system in R² is optionally substituted by at least one substituent selected from halogen, nitro, C₂-C₆ alkenyl, C₁-C₆ alkoxy, C₁-C₆ alkoxycarbonyl, C₁-C₆ alkylcarbonyl, phenylcarbonyl, C₁-C₆ alkyl, isoxazolyl, pyrazolyl, -NR¹²R¹³, -C(O)NR¹⁴R¹⁵, -NHC(O)NR¹⁶R¹⁷, and -NHC(O)R²³.

Each R³ independently represents a C₁-C₆, preferably C₁-C₄, alkyl (e.g. methyl, ethyl, n-propyl, isopropyl, n-butyl, isobutyl, tert-butyl, n-pentyl or n-hexyl), C₁-C₆, preferably C₁-C₄, alkoxycarbonyl (e.g. methoxycarbonyl or ethoxycarbonyl), -CH₂OH or carboxyl group. In one embodiment, each R³ independently represents a methyl, methoxycarbonyl, ethoxycarbonyl, -CH₂OH or carboxyl group.

R⁶ and R⁷ each independently represent a hydrogen atom or a C₁-C₆, preferably C₁-C₄, alkyl group (e.g. methyl, ethyl, n-propyl, isopropyl, n-butyl, isobutyl, tert-butyl, n-pentyl or n-hexyl), or R⁶ and R⁷ together with the nitrogen atom to which they are attached form a 4- to 7-membered saturated heterocycle (such as pyrrolidinyl or piperidinyl).

R⁸ and R⁹ each independently represent a hydrogen atom or a C₁-C₆, preferably C₁-C₄, alkyl group (e.g. methyl, ethyl, n-propyl, isopropyl, n-butyl, isobutyl, tert-butyl, n-pentyl or n-hexyl) optionally substituted by at least one (e.g. one or two) C₁-C₆, preferably C₁-C₄, alkoxycarbonyl.

R¹⁰ represents a hydrogen atom or a C₁-C₆, preferably C₁-C₄, alkyl group (e.g. methyl, ethyl, n-propyl, isopropyl, n-butyl, isobutyl, tert-butyl, n-pentyl or n-hexyl).

R¹¹ represents a hydrogen atom, or a C₁-C₆, preferably C₁-C₄, alkyl group (e.g. methyl, ethyl, n-propyl, isopropyl, n-butyl, isobutyl, tert-butyl, n-pentyl or n-hexyl) optionally substituted by at least one substituent (e.g. one or two substituents independently) selected from carboxyl, C₁-C₆, preferably C₁-C₄, alkoxy and C₁-C₆, preferably C₁-C₄, alkoxycarbonyl.

R¹² and R¹³ each independently represent a hydrogen atom, or a C₁-C₆, preferably C₁-C₄, alkyl group (e.g. methyl, ethyl, n-propyl, isopropyl, n-butyl, isobutyl, tert-butyl, n-pentyl or n-hexyl) optionally substituted by at least one substituent (e.g. one or two substituents independently) selected from phenyl, carboxyl and C₁-C₆, preferably C₁-C₄, alkoxycarbonyl (e.g. methoxycarbonyl or ethoxycarbonyl), or R¹² and R¹³ together with the nitrogen atom to which they are attached form a 4- to 7-membered saturated heterocycle (such as pyrrolidinyl or piperidinyl).

R¹⁴ and R¹⁵ each independently represent a hydrogen atom, a C₃-C₆ cycloalkyl (cyclopropyl, cyclobutyl, cyclopentyl or cyclohexyl) group, or a C₁-C₆, preferably C₁-C₄, alkyl group (e.g. methyl, ethyl, n-propyl, isopropyl, n-butyl, isobutyl, tert-butyl, n-pentyl or n-hexyl) optionally substituted by at least one substituent (e.g. one or two substituents independently) selected from carboxyl and C₁-C₆, preferably C₁-C₄, alkoxycarbonyl (e.g. methoxycarbonyl or ethoxycarbonyl), or R¹⁴ and R¹⁵ together with the nitrogen atom to which they are attached form a 4- to 7-membered saturated heterocycle (such as pyrrolidinyl or piperidinyl).

R¹⁶ and R¹⁷ each independently represent a hydrogen atom, or a C₁-C₆, preferably C₁-C₄, alkyl group (e.g. methyl, ethyl, n-propyl, isopropyl, n-butyl, isobutyl, tert-butyl, n-pentyl or n-hexyl) optionally substituted by at least one substituent (e.g. one or two substituents independently) selected from carboxyl and C₁-C₆, preferably C₁-C₄, alkoxycarbonyl (e.g. methoxycarbonyl or ethoxycarbonyl), or R¹⁶ and R¹⁷ together with the nitrogen atom to which they are attached form a 4- to 7-membered saturated heterocycle (such as pyrrolidinyl or piperidinyl).

R¹⁸ and R¹⁹ each independently represent a hydrogen atom, or a C₁-C₆, preferably C₁-C₄, alkyl group (e.g. methyl, ethyl, n-propyl, isopropyl, n-butyl, isobutyl, tert-butyl, n-pentyl or n-hexyl) optionally substituted by at least one substituent (e.g. one or two substituents independently) selected from carboxyl and C₁-C₆, preferably C₁-C₄, alkoxycarbonyl (e.g. methoxycarbonyl or ethoxycarbonyl), or R¹⁸ and R¹⁹ together with the nitrogen atom to which they are attached form a 4- to 7-membered saturated heterocycle (such as pyrrolidinyl or piperidinyl).

R²⁰ and R²¹ each independently represent a hydrogen atom, or a C₁-C₆, preferably C₁-C₄, alkyl group (e.g. methyl, ethyl, n-propyl, isopropyl, n-butyl, isobutyl, tert-butyl, n-pentyl or n-hexyl) optionally substituted by at least one substituent (e.g. one or two substituents independently) selected from carboxyl and C₁-C₆, preferably C₁-C₄, alkoxycarbonyl (e.g. methoxycarbonyl or ethoxycarbonyl), or R²⁰ and R²¹ together with the nitrogen atom to which they are attached form a 4- to 7-membered saturated heterocycle (such as pyrrolidinyl or piperidinyl).

R²² represents a hydrogen atom, or a C₁-C₆, preferably C₁-C₄, alkyl group (e.g. methyl, ethyl, n-propyl, isopropyl, n-butyl, isobutyl, tert-butyl, n-pentyl or n-hexyl) optionally substituted by at least one substituent (e.g. one or two substituents independently) selected from carboxyl and C₁-C₆, preferably C₁-C₄, alkoxycarbonyl (e.g. methoxycarbonyl or ethoxycarbonyl).

R²³ represents a group C₁-C₆, preferably C₁-C₅, alkyl group (e.g. methyl, ethyl, n-propyl, isopropyl, n-butyl, isobutyl, tert-butyl, n-pentyl or n-hexyl), C₂-C₆, preferably C₂-C₄, alkenyl, C₃-C₆ cycloalkyl (cyclopropyl, cyclobutyl, cyclopentyl or cyclohexyl), adamantyl, C₅-C₆ cycloalkenyl, phenyl or a saturated or unsaturated 5- to 10-membered heterocyclic ring system comprising at least one heteroatom (e.g. one, two, three or four heteroatoms independently) selected from nitrogen, oxygen and sulphur, each group being optionally substituted by at least one substituent (e.g. one, two, three or four substituents independently) selected from nitro, hydroxyl, oxo, halogen (e.g. fluorine, chlorine, bromine or iodine), carboxyl, C₁-C₆, preferably C₁-C₄, alkyl (e.g. methyl, ethyl, n-propyl, isopropyl, n-butyl, isobutyl, tert-butyl, n-pentyl or n-hexyl), C₁-C₆, preferably C₁-C₄, alkoxy (e.g. methoxy, ethoxy, n-propoxy or n-butoxy), C₁-C₆, preferably C₁-C₄, alkylthio (e.g. methylthio or ethylthio), C₁-C₆, preferably C₁-C₄, alkylcarbonyl (e.g. methylcarbonyl, ethylcarbonyl, n-propylcarbonyl, isopropylcarbonyl, n-butylcarbonyl, n-pentylcarbonyl or n-hexylcarbonyl), C₁-C₆, preferably C₁-C₄, alkoxycarbonyl (e.g. methoxycarbonyl or ethoxycarbonyl), phenyl and -NHC(O)-R²⁴.

The saturated or unsaturated 5- to 10-membered heterocyclic ring system may be monocyclic or polycyclic (fused or otherwise), e.g. bicyclic, and may comprise up to four heteroatoms independently selected from nitrogen, oxygen and sulphur. Examples of ring systems that may be used include pyrrolidinyl, piperidinyl, pyrazolyl, thiazolidinyl, thienyl, isoxazolyl, thiadiazolyl, pyrrolyl, furanyl, thiazolyl, indolyl, quinolinyl, benzimidazolyl, triazolyl, tetrazolyl and pyridinyl.

R²⁴ represents a C₁-C₆, preferably C₁-C₄, alkyl group (e.g. methyl, ethyl, n-propyl, isopropyl, n-butyl, isobutyl, tert-butyl, n-pentyl or n-hexyl), amino or phenyl group.

In an embodiment of the invention,
m is l;
R¹ represents halogen;
X represents a group CH₂ or (CH₂)₂;
Y represents an oxygen atom or a group CH₂;
R² represents an unsaturated 5- to 10-membered ring system which may comprise at least one ring heteroatom (e.g. one or two ring heteroatoms independently) selected from nitrogen, oxygen and sulphur, the ring system being optionally substituted by at least one substituent (e.g. one, two or three substituents independently) selected from halogen, nitro, C₂-C₆ alkenyl, C₁-C₆ alkoxy, C₁-C₆ alkoxycarbonyl, C₁-C₆ alkylcarbonyl, phenylcarbonyl, C₁-C₆ alkyl, isoxazolyl, pyrazolyl, -NR¹²R¹³, -C(O)NR¹⁴R¹⁵, -NHC(O)NR¹⁶R¹⁷, and -NHC(O)R²³;
n is 0;
R¹² and R¹³ each independently represent a hydrogen atom, or a C₁-C₆ alkyl group optionally substituted by at least one phenyl;
R¹⁴ and R¹⁵ each independently represent a hydrogen atom, a C₃-C₆ cycloalkyl group or a C₁-C₆ alkyl group;
R¹⁶ and R¹⁷ each independently represent a hydrogen atom or a C₁-C₆ alkyl group; and
R²³ represents a C₁-C₆ alkyl group or a phenyl group.

Examples of compounds of the invention include:
N-[2-({4-(4- fluorophenyl)-4-oxobutyl]-2-morpholinyl}methoxy)phenyl]acetamide,
N-[2-({3-(4-Fluorophenyl)-3-oxopropyl]-2-morpholinyl}methoxy)phenyl]acetamide,
N-[2-({1-[4-(4-Fluorophenyl)-4-oxobutyl)-3-piperidinyl}methoxy)phenyl]acetamide,
N-[2-({4-(4-Fluorophenyl)-4-oxobutyl]-2-morpholinyl}methoxy)phenyl]acetamide,
1-(4-Fluorophenyl)-4-{2-[3-methoxyphenoxy)methyl]-4-morpholinyl}-1-butanone,
N-[2-({(3R)-1-[4-(4-Fluorophenyl)-4-oxobutyl]-3-piperidinyl}methoxy)phenyl]-acetamide,
N-[2-({(3S)-1-[4-(4-Fluorophenyl)-4-oxobutyl]-3-piperidinyl}methoxy)phenyl]-acetamide,
N-[2-({1-[4-(4-Fluorophenyl)-4-oxobutyl]-3-piperidinyl}methoxy)phenyl]-benzamide,
4-{3-[(2-Acetylphenoxy)methyl]-1-piperidinyl}-1-(4-fluorophenyl)-1-butanone,
4-{3-[(3-Benzoylphenoxy)methyl]-1-piperidinyl}-1-(4-fluorophenyl)-1-butanone,
4-{3-[(1,3-Benzothiazol-2-yloxy)methyl]-1-piperidinyl}-1-(4-fluorophenyl)-1-butanone,
1-(4-Fluorophenyl)-4-{3-[(8-quionolinyloxy)methyl]-1-piperidinyl}-1-butanone,
1-(4-Fluorophenyl)-4-(3-{[(2-methyl-8-quinolinyl)oxy]methyl}-1-piperidinyl)-1-butanone,
1-(4-Fluorophenyl)-4-{3-[(4-quinozolinyloxy)methyl]-1-piperidinyl}-1-butanone,
1-(4-Fluorophenyl)-4-{3-[(1-naphthyloxy)methyl]-1-piperidinyl}-1-butanone,
1-(4-Fluorophenyl)-4-(3-{[2-(5-isoxazolyl)phenoxy]methyl}-1-piperidinyl)-1-butanone,
4-(3-{[4-Chloro-2-(1H-pyrazol-5-yl)phenoxy]methyl}-1-piperidinyl)-1-(4-fluorophenyl)-1-butanone,
N-Cyclopropyl-2-({1-[4-(4-fluorophenyl)-4-oxobutyl]-3-piperidinyl}methoxy)-benzamide,
Methyl 2-({1-[4-(4-fluorophenyl)-4-oxobutyl]-3-piperidinyl}methoxy)benzoate,
2-({1-[4-(4-Fluorophenyl)-4-oxobutyl]-3-piperidinyl}methoxy)-N-methylbenzamide,
1-(4-Fluorophenyl)-4-[3-({2-[(E)-1-propenyl]phenoxy}methyl)-1-piperidinyl]-1-butanone,
4-{3-[3,5-Difluorophenoxy)methyl]-1-piperidinyl}-1-(4-fluorophenyl)-1-butanone,
4-[3-[(4-Chloro-2-isopropyl-5-methylphenoxy)methyl]-1-piperidinyl}-1-(4-fluorophenyl)-1-butanone,
4-{3-[3,4-Dinitrophenoxy)methyl]-1-piperidinyl}-1-(4-fluorophenyl)-1-butanone,
1-(4-Fluorophenyl)-4-{3-[(2-isopropoxyphenoxy)methyl]-1-piperidinyl}-1-butanone,
1-(4-Fluorophenyl)-4-{3-[(2-propionylphenoxy)methyl]-1-piperidinyl}-1-butanone,
4-{3-[(2-Butyrylphenoxy)methyl]-1-piperidinyl}-1-(4-fluorophenyl)-1-butanone,
1-(4-Fluorophenyl)-4-(3-{[(3-methyl-1,2-benzisoxazol-4-yl)-oxy]methyl}-1-piperidinyl)-1-butanone,
N-[2-({1-[4-(4-Fluorophenyl)-4-oxobutyl]-3-piperidinyl}methoxy)phenyl]urea,
5-Chloro-2-({1-[4-(4-fluorophenyl)-4-oxobutyl]-3-piperidinyl}methoxy)benzamide,
N-Ethyl-N'-[2-({1-[4-(4-fluorophenyl)-4-oxobutyl]-3-piperidinyl}methoxy)-phenyl]urea,
N'-[2-({1-[4-(4-Fluorophenyl)-4-oxobutyl]-3-piperidinyl}methoxy)phenyl]-N,N-dimethylurea,
4-(3-{[2-(Benzylamino)phenoxy]methyl}-1-piperidinyl)-1-(4-fluorophenyl)-1-butanone,
1-(4-Fluorophenyl)-4-(3-{[(2-methyl-1,3-benzoxazol-4-yl)oxy]methyl}-1-piperidinyl)-1-butanone,
and pharmaceutically acceptable salts and solvates of any one thereof.

The present invention further provides a process for the preparation of a compound of formula (I) as defined above which comprises reacting a compound of formula 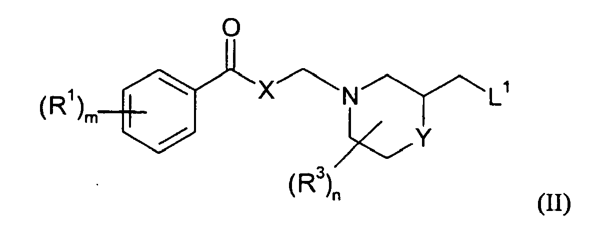 wherein L¹ represents a leaving group (e.g. nitrobenzenesulphonate or
p-toluenesulphonate) and m, n, X, Y, R¹ and R³ are as defined in formula (I), with a compound of formula

R² - OH (II)

wherein R² is as defined in formula (I);
and optionally thereafter forming a pharmaceutically acceptable salt or solvate of the compound of formula (I) obtained.

Compounds of formula (II) in which the leaving group, L¹, is a 3-nitrobenzenesulphonate group may conveniently be prepared according to the reaction scheme shown on the following page. Compounds of formula (II) in which the leaving group, L¹, is a p-toluenesulphonate group may be prepared in a similar manner using p-toluenesulphonyl chloride in place of 3-nitrobenzenesulphonyl chloride in the final step.

Compounds of formula (III) are either commercially available, are well known in the literature or may be prepared easily using known techniques.

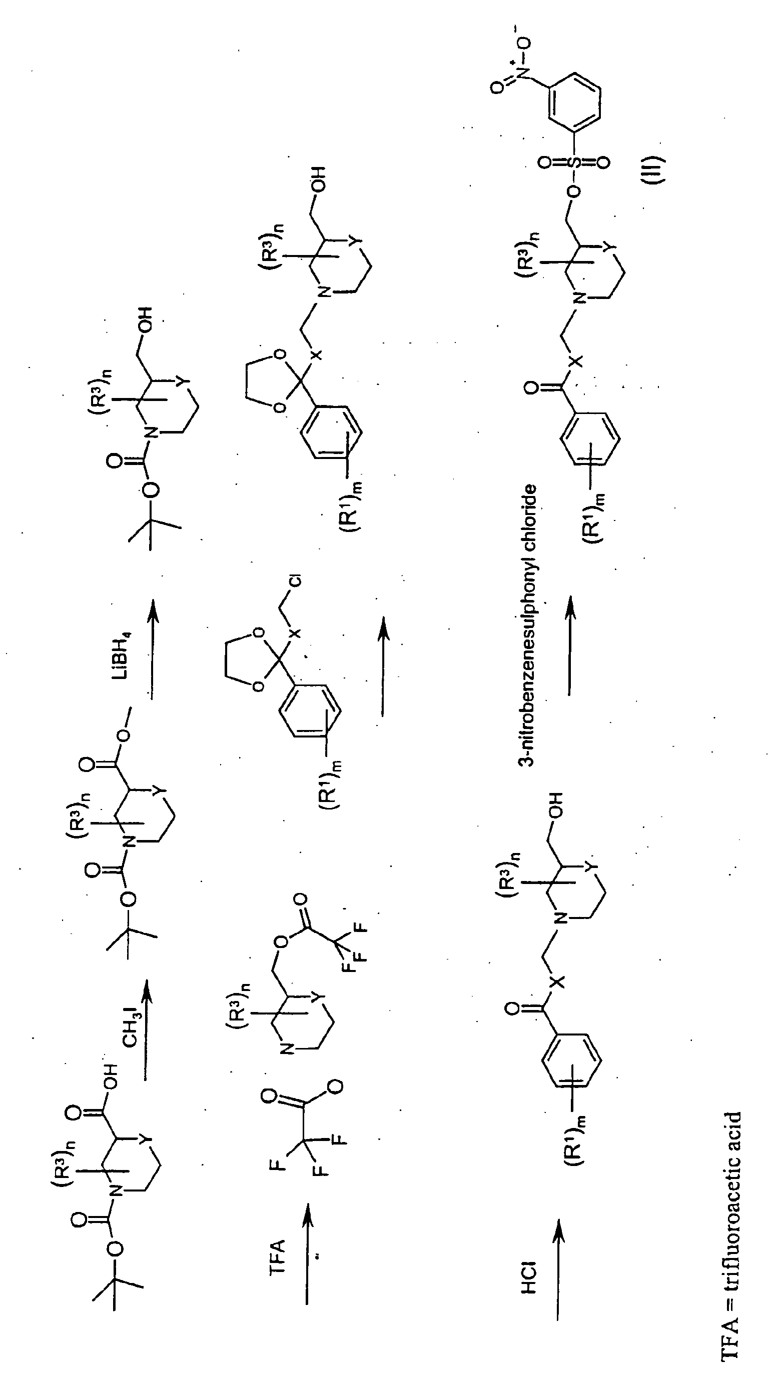

It will be appreciated by those skilled in the art that in the process of the present invention certain functional groups such as hydroxyl or amino groups in the starting reagents or intermediate compounds may need to be protected by protecting groups. Thus, the preparation of the compounds of formula (I) may involve, at an appropriate stage, the removal of one or more protecting groups.

The protection and deprotection of functional groups is described in 'Protective Groups in Organic Chemistry', edited by J.W.F. McOmie, Plenum Press (1973) and 'Protective Groups in Organic Synthesis', 2nd edition, T.W. Greene and P.G.M. Wuts, Wiley-Interscience (1991).

The compounds of formula (I) above may be converted to a pharmaceutically acceptable salt or solvate thereof, preferably an acid addition salt such as a hydrochloride, hydrobromide, phosphate, acetate, fumarate, maleate, tartrate, citrate, oxalate, methanesulphonate orp-toluenesulphonate.

Compounds of formula (I) are capable of existing in stereoisomeric forms. It will be understood that the invention encompasses the use of all geometric and optical isomers (including atropisomers) of the compounds of formula (I) and mixtures thereof including racemates. The use of tautomers and mixtures thereof also form an aspect of the present invention. Enantiomerically pure forms are particularly desired.

The compounds of formula (I) have activity as pharmaceuticals, in particular as modulators of chemokine receptor (especially MIP-1α chemokine receptor) activity, and may be used in the treatment of autoimmune, inflammatory, proliferative and hyperproliferative diseases and immunologically-mediated diseases including rejection of transplanted organs or tissues and Acquired Immunodeficiency Syndrome (AIDS).

Examples of these conditions are:
(1) **(the respiratory tract)** airways diseases including chronic obstructive pulmonary disease (COPD) such as irreversible COPD; asthma, such as bronchial, allergic, intrinsic, extrinsic and dust asthma, particularly chronic or inveterate asthma (e.g. late asthma and airways hyper-responsiveness); bronchitis; acute, allergic, atrophic rhinitis and chronic rhinitis including rhinitis caseosa, hypertrophic rhinitis, rhinitis purulenta, rhinitis sicca and rhinitis medicamentosa; membranous rhinitis including croupous, fibrinous and pseudomembranous rhinitis and scrofoulous rhinitis; seasonal rhinitis including rhinitis nervosa (hay fever) and vasomotor rhinitis; sarcoidosis, farmer's lung and related diseases, fibroid lung and idiopathic interstitial pneumonia;
(2) **(bone and joints)** rheumatoid arthritis, seronegative spondyloarthropathies (including ankylosing spondylitis, psoriatic arthritis and Reiter's disease), Behcet's disease, Sjogren's syndrome and systemic sclerosis;
(3) **(skin)** psoriasis, atopical dermatitis, contact dermatitis and other eczmatous dermitides, seborrhoetic dermatitis, Lichen planus, Pemphigus, bullous Pemphigus, Epidermolysis bullosa, urticaria, angiodermas, vasculitides, erythemas, cutaneous eosinophilias, uveitis, Alopecia areata and vernal conjunctivitis;
(4) **(gastrointestinal tract)** Coeliac disease, proctitis, eosinopilic gastro-enteritis, mastocytosis, Crohn's disease, ulcerative colitis, food-related allergies which have effects remote from the gut, e.g., migraine, rhinitis and eczema;
(5) **(other tissues and systemic disease)** multiple sclerosis, atherosclerosis, Acquired Immunodeficiency Syndrome (AIDS), lupus erythematosus, systemic lupus, erythematosus, Hashimoto's thyroiditis, myasthenia gravis, type I diabetes, nephrotic syndrome, eosinophilia fascitis, hyper IgE syndrome, lepromatous leprosy, sezary syndrome and idiopathic thrombocytopenia pupura;
(6) **(allograft rejection)** acute and chronic following, for example, transplantation of kidney, heart, liver, lung, bone marrow, skin and cornea; and chronic graft versus host disease;
(7) cancers, especially non-small cell lung cancer (NSCLC) and squamous sarcoma;
(8) diseases in which angiogenesis is associated with raised chemokine levels; and
(9) cystic fibrosis, stroke, re-perfusion injury in the heart, brain, peripheral limbs and sepsis.

Thus, the present invention provides a compound of formula (I), or a pharmaceutically-acceptable salt or solvate thereof, as hereinbefore defined for use in therapy.

In a further aspect, the present invention provides the use of a compound of formula (I), or a pharmaceutically acceptable salt or solvate thereof, as hereinbefore defined in the manufacture of a medicament for use in therapy.

In the context of the present specification, the term "therapy" also includes "prophylaxis" unless there are specific indications to the contrary. The terms "therapeutic" and "therapeutically" should be construed accordingly.

The invention also provides a use of a compound of formula (I) in the manufacture of a medicament for treating an inflammatory disease which comprises administering to a patient in need thereof a therapeutically effective amount of a compound of formula (I), or a pharmaceutically acceptable salt or solvate thereof, as hereinbefore defined.

The invention still further provides a use of a compound of formula (I) in the manufacture of a medicament for treating an airways disease which comprises administering to a patient in need thereof a therapeutically effective amount of a compound of formula (I), or a pharmaceutically acceptable salt or solvate thereof, as hereinbefore defined.

For the above-mentioned therapeutic uses the dosage administered will, of course, vary with the compound employed, the mode of administration, the treatment desired and the disorder indicated. The daily dosage of the compound of formula (I) may be in the range from 0.001 mg/kg to 30 mg/kg.

The compounds of formula (I) and pharmaceutically acceptable salts and solvates thereof may be used on their own but will generally be administered in the form of a pharmaceutical composition in which the formula (I) compound/salt/solvate (active ingredient) is in association with a pharmaceutically acceptable adjuvant, diluent or carrier. Depending on the mode of administration, the pharmaceutical composition will preferably comprise from 0.05 to 99 %w (per cent by weight), more preferably from 0.05 to 80 %w, still more preferably from 0.10 to 70 %w, and even more preferably from 0.10 to 50 %w, of active ingredient, all percentages by weight being based on total composition.

The present invention also provides a pharmaceutical composition comprising a compound of formula (I), or a pharmaceutically acceptable salt or solvate thereof, as hereinbefore defined, in association with a pharmaceutically acceptable adjuvant, diluent or carrier.

The invention further provides a process for the preparation of a pharmaceutical composition of the invention which comprises mixing a compound of formula (I), or a pharmaceutically acceptable salt or solvate thereof, as hereinbefore defined, with a pharmaceutically acceptable adjuvant, diluent or carrier.

The pharmaceutical compositions may be administered topically (e.g. to the skin or to the lung and/or airways) in the form, e.g., of creams, solutions, suspensions, heptafluoroalkane aerosols and dry powder formulations; or systemically, e.g. by oral administration in the form of tablets, capsules, syrups, powders or granules; or by parenteral administration in the form of solutions or suspensions; or by subcutaneous administration; or by rectal administration in the form of suppositories; or transdermally.

The invention will now be further explained by reference to the following illustrative examples, in which ¹H NMR spectra were recorded on Varian Unity Inova 400. The central solvent peak of chloroform-*d* (δ_{H} 7.27 ppm) were used as internal standard. Low resolution mass spectra and accurate mass determination were recorded on a Hewlett-Packard 1100 LC-MS system equipped with APCI /ESI ionisation chambers. All solvents and commercial reagents were laboratory grade and used as received.
The nomenclature used for the compounds was generated with ACD/IUPAC Name Pro.

### Example 1

### N-[2-({4-(4-fluorophenyl)-4-oxobutyl]-2-morpholinyl}methoxy)phenyl]acetamide

### (i) 4-(tert-Butyl) 2-methyl 2,4-morpholinedicarboxylate

Methyl iodide (9.38 ml, 150 mmol) was added to a suspension of 4-(*tert*-butoxycarbonyl)-2-morpholinecarboxylic acid (14.5 g, 62.6 mmol) and dry potassium carbonate (17.3 g, 125 mmol) in dry dimethylformamide (DMF) (360 ml). The mixture was stirred over night, filtered through Celite and concentrated. The residue was partitioned between dichloromethane and water. The organic phase was dried over magnesium sulfate and concentrated to give 22 g of crude product.

¹H-NMR (400 MHz, CDCl₃): δ 4.07 (2H, dd), 3.99 (1H, 2m), 3.77 (3H, s), 3.73 (1H, m), 3.55 (1H, m), 3.07 (2H, m), 1.45 (9H, s).

### (ii) tert-Butyl 2-(hydroxymethyl)-4-morpholinecarboxylate

The crude product from step (i) (62.6 mmol) was dissolved in dry tetrahydrofuran (THF) (100 ml) and added dropwise at 0°C to a suspension of lithium borohydride (2.50 g, 115 mmol) in dry THF (100 ml). The mixture was allowed to attain room temperature over night. Water (10 ml) was added and after stirring for 1 h the mixture was concentrated.

The residue was partitioned between ethyl acetate and water. The organic phase was washed with 0.5 M hydrochloric acid, saturated sodium hydrogen carbonate and water. Drying over magnesium sulfate and concentration gave the title compound as a crude product (13.3 g).

¹H-NMR (400 MHz, CDCl₃): δ 3.88 (3H, m), 3.46-3.72 (4H, m), 2.93 (1H, m), 2.75 (1H, m), 2.09 (1H, m), 1.46 (9H, s).

### (iii) 2-Morpholinylmethyl 2,2,2 trifluoroacetate (trifluoroacetic acid salt)

*Tert*-butyl 2-(hydroxymethyl)-4-morpholinecarboxylate, obtained in step (ii) (5.13 g, 23.61 mmol) was treated with trifluoroacetic acid (20 mL) in dichloromethane (50 mL) at room temperature for 3 h. The volatiles were removed in vacuo to give subtitled compound (yield 7.6 g).

¹H-NMR (DMSOd₆, 400 MHz): δ 9.25 (br.,s, 2H); 3.86 (dd, J = 3.3, 12.6 Hz, 1H); 3.62 (m, 2H); 3.39 (m, 2H); 3.19 (m, 2H); 2.96 (t, J = 11.2 Hz, 1H); 2.76 (t,J = 11.2 Hz, 1H).

### (iv) (4{3-[2-(4-fluorophenyl)-1,3-dioxolan-2-yl]propyl}-2-morpholinyl)methanol

A mixture of 2-morpholinylmethyl 2,2,2 trifluoroacetate (trifluoroacetic acid salt) obtained from step (iii) (700 mg, 2.13 mmol), 2-(3-chloropropyl)-2-(4-fluorophenyl)-1,3-dioxolane (624 mg, 2.55 mmol), NaI (382.2 mg, 2.55 mmol) and potassium carbonate (K₂CO₃) (1.40 g) in acetonitrile was stirred at reflux temperature overnight. The reaction mixture was cooled to room temperature and partitioned between ethylacetate and water. The organic layer was dried over sodium sulphate (Na₂SO₄), filtered and concentrated. The residue was purifified by silica gel flash chromatography to give subtitled compound (600 mg).

¹H-NMR (CDCl₃, 400 MHz): δ 7.41 (m, 2H); 7.00 (m, 2H); 4.00 (m, 1H); 3.90 (m, 1H); 3.79-3.50 (m, 6H); 2.71 (t, J = 10.0 Hz, 2H); 2.39 (m, 2H); 2.25 (t, J = 10.1 Hz, 1H); 2.00 (t, J = 9.3 Hz, 1H); 1.88 (m, 2H); 1.58 (m,2H).
APCI-MS: m/z 326 (MH⁺).

### (v) 1-(4-Fluorophenyl)-4-[2-(hydroxymethyl)-4-morpholinyl]-1-butanone

The compound (4{3-[2-(4-fluorophenyl)-1,3-dioxolan-2-yl]propyl}-2-morpholinyl)methanol obtained from step (iv) (600 mg, 1.84 mmol) was treated with aqueous 2 M HCl (10 mL) in methanol (10 mL) at reflux temperature for 3 h. The volatiles were removed in vacuo, residue was dissolved in chloroform, washed with saturated aqueous sodium hydrogen carbonate (NaHCO₃) and water (H₂O) successively. The organic layer was dried over sodium sulphate (Na₂SO₄), filtered and concentrated to give the subtitled compound (497 mg).

¹H-NMR (CDCl₃, 400 MHz): δ 8.00 (m, 2H); 7.13(m, 2H); 3.88 (m, 1H); 3.74-3.50 (m, 4H); 3.00 (t, J = 7.0 Hz, 2H); 2.73 (t, J = 10.7 Hz, 2H); 2.46 (t, J = 5.5 Hz, 2H); 2.20 (t, J = 10.4 Hz, 1H); 2.10-1.90 (m, 4H).
APCI-MS: m/z 282 (MH⁺).

### (vi) {4-[4-Fluorophenyl)-4-oxobutyl]-2-morpholinyl}methyl-3-nitrobenzenesulfonate

To a solution of 1-(4-fluorophenyl)-4-[2-(hydroxymethyl)-4-morpholinyl]-1-butanone obtained from step (v) (490 mg, 1.74 mmol) in dichloromethane (CH₂Cl₂) (8 mL) was added triethylamine (Et₃N) (0.871 mL) followed by 3-nitrobenzenesulfonyl chloride (463 mg, 2.09 mmol) and the reaction mixture was stirred at room temperature overnight. The volatiles were removed in vacuo and the residue dissolved in chloroform, washed with saturated aqueous sodium hydrogen carbonate (NaHCO₃) solution. The organic layer was dried over sodium sulphate (Na₂SO₄), filtered and concentrated. The residue was purified by silica gel flash chromatography to give the subtitled compound (460 mg).

¹H-NMR (CDCl₃, 400 MHz): δ 8.78 (m, 1H); 8.49 (m, 1H); 8.23 (m, 1H); 7.99 (m, 2H); 7.78 (t, J = 7.9 Hz, 1H); 7.12 (m, 2H); 4.15 (d, J = 4.8 Hz, 2H); 3.70 (m, 2H); 3.49 (m, 1H); 2.99 (t, J = 7.0 Hz, 2H); 2.70 (m, 2H); 2.41 (m, 2H); 2.20 (t, J = 9.0 Hz, 1H); 1.98 (m, 3H).
APCI-MS: m/z 467 (MH⁺).

### (vii)N-[2-({4-(4-Fluorophenyl)-4-oxobutyl]-2-morpholinyl}methoxy)phenyl]acetamide

A mixture of {4-[4-fluorophenyl)-4-oxobutyl]-2-moipholinyl}methyl-3-nitrobenzenesulfonate (0.2 mL, 0.043 M solution in DMF), 2-acetamidophenol (0.2 mL, 0.043 M solution in DMF) and potassium carbonate (K₂CO₃) (35 mg) was shaken at 65°C for 5 h. The volatiles were removed in vacuo and dissolved in dichloromethane (CH₂Cl₂) and liquid-liquid extraction was performed, filtered and concentrated to give the titled compound.
APCI-MS: m/z 414 (MH⁺).

### Example 2

### N-(2-({3-(4-Fluorophenyl)-3-oxopropyl]-2-morpholinyl}methoxy)phenyl]acetamide

### (i) 1-(4-Fluorophenyl)-3-[2-(hydroxymethyl-4-morpholinyl]-1-propanone

The reaction was performed as described in Example 1, step (iv) using 2-morpholinylmethyl 2,2,2 trifluoroacetate (trifluoroacetic acid salt) (1.2 g, 3.66 mmol), 3-chloro-1-(4-fluorophenyl)-1-propanone (683 mg, 3.66 mmol), triethylamine (2.54 mL, 18.3 mmol), acetonitrile (10 mL) to give the subtitled compound (700 mg).

¹H-NMR (CDCl₃, 400 MHz): δ 7.99 (m, 2H); 7.13 (m, 2H); 3.92 (m, 1H); 3.76-3.53 (m, 4H); 3.19 (t, J = n7.0 Hz,2H); 2.93-2.70 (m, 4H); 2.28 (m, 1H); 2.11 (t, J = 10.7 Hz, 1H). APCI-MS: m/z 268 (MH⁺).

### (ii) {4-[3-(4-Fluorophenyl)-3-oxopropyl]-2-morpholinyl}methyl-3-nitrobenzenesulfonate

The reaction was performed as described in Example 1, step (vi) using 1-(4-fluorophenyl)-3-[2-(hydroxymethyl-4-morpholinyl]-1-propanone obtained in step (i) above (691 mg, 2.58 mmol), 3-nitrobenzenesulfonyl chloride (687 mg, 3.10 mmol), and triethylamine (1.29 mL, 9.30 mmol) in dichloromethane (10 mL) to give the subtitled compound (900 mg). ). ¹H-NMR (CDCl₃, 400 MHz): δ 8.38 (m, 2H); 8.16 (m, 2H); 7.96 (m, 2H); 7.14 (m, 2H); 4.14 (d, J = 5.1 Hz 2H); 3.77 (d, J = 10.7 Hz, , 2H); 3.55 (t, J = 11.0 Hz, 1H); 3.16 (t, J = 6.6 Hz, 2H); 2.81 (m, 3H); 2.68 (m, 1H); 2.20 (t, J 10.2 Hz, 1H); 2.01 (t, J = 9.6 Hz, 1H). APCI-MS: m/z 453 (MH⁺).

### (iii) N-[2-({3-(4-Fluorophenyl)-3-oxopropyl]-2-morpholinyl}methoxy)phenyl]-acetamide

The reaction was performed as described in Example 1, step (vii) to give the titled compound.

APCI-MS: m/z 400 (MH⁺).

### Example 3

### N-[2-({1-[4-(4-Fluorophenyl)-4-oxobutyl)-3-piperidinyl}methoxy)phenyl]acetamide

### (i) (1-{3-[2-(4-Fluorophenyl)-1,3-dioxolan-2-yl]propyl}-3-piperidinyl)methanol

A mixture of 2-(3-chloropropyl)-2-(4-fluorophenyl)-1,3-dioxolane (1.17 g, 4.80 mmol), 3-piperidinylmethanol (460.72 mg, 4.0 mmol), sodium iodide (NaI) (719.5 mg, 4.8 mmol) and potassium carbonate (K₂CO₃) (1.99 g) in acetonitrile was stirred at reflux temperature overnight. The reaction mixture was partitioned between ethylacetate and water (H₂O), organic layer dried over sodium sulphate (Na₂SO₄), filtered, concentrated and the residue was purified by silica gel flash chromatography to give the subtitle compound (1.0 g).

¹H-NMR (CDCl₃, 400 MHz): δ 7.40 (m, 2H); 7.00 (m, 2H); 4.08 (m, 2H); 3.77 (m, 2H); 3.63 (dd, J = 5.0, 10.8 Hz, 1H); 3.53 (dd, J = 6.1, 10.7 Hz, 1H); 2.95 (d, J = 10.1 Hz, 2H); 2.74 (m, 2H); 2.50 (t, J = 7.7 Hz, 2H); 2.25 (m, 4H); 2.00 (m, 2H); 1.90-1.60 (m, 6H). APCI-MS: m/z 324 (MH⁺).

### (ii) 1-(4-Fluorophenyl)-4-[3-(hydroxymethyl)-1-piperidinyl]-1-butanone

The compound (1-{3-[2-(4-fluorophenyl)-1,3-dioxolan-2-yl]propyl}-3-piperidinyl)methanol obtained from step (i) above (1.0 g, 3.09 mmol) was treated with 2M aqueous hydrochloric acid (HCl) (5 mL) in methanol (7 mL) at reflux temperature for 3 h. The volatiles were removed in vacuo and residue dissolved in trichloromethane (CHCl₃), washed successively with saturated aqueous potassium carbonate (K₂CO₃) and water (H₂O). The organic layer was dried over sodium sulphate (Na₂SO₄), filtered, concentrated to give the subtitle compound (670 mg).

¹H-NMR (CDCl₃, 400 MHz): δ 8.00 (m, 2H); 7.12 (m, 2H); 3.65 (m, 1H); 3.51 (m, 1H); 2.99 (m, 2H); 2.79 (br. d, J = 9.4 Hz, 1 H); 2.62 (m, 1H); 2.45 (t, J = 7.0 Hz, 2H); 2.20 (t. J = 8.9 Hz, 1H); 2.09 (t, 1H); 1.95 (m, 2H); 1.82-1.50 (m, 4H). ).
APCI-MS: m/z 280 (MH⁺).

### (iii) {1-[4-(4-fluorophenyl)-4oxobutyl]-3-piperidinyl}methylbenzenesulfonate

To a solution of 1-(4-fluorophenyl)-4-[3-(hydroxymethyl)-1-piperidinyl]-1-butanone (8,54 g, 30.56 mmol), in dichloromethane (CH₂Cl₂) (100 mL) was added triethylamine (Et₃N) (22 mL) followed by p-toluenesulfonyl chloride (7.28 g, 38.2 mmol) and the reaction mixture kept on stirring at room temperature for overnight. The volatiles were removed in vacuo, residue dissolved in chloroform; washed successively with saturated aqueous sodium hydrogen carbonate (NaHCO₃) and water. The organic layer was dried over sodium sulphate (Na₂SO₄), filtered, concentrated and the residue was purified by silica gel flash chromatography to give the subtitle compound (9.0 g).

¹H-NMR (CDCl₃, 400 MHz): δ 8.00 (m, 2H); 7.78 (d, J = 8.3 Hz,2H); 7.35 (d, J = 8.0 Hz, 2H); 7.15 (m, 2H); 3.90 (m, 2H); 2.97 (t, J = 7.0 Hz, 2H); 2.75 (m, 2H); 2.45 (s, 3H); 2.38 (t, J = 7.0 Hz, 2H); 1.92 (m, 5H); 1.62 (m, 2H), 1.48 (m, 1H), 1.01 (m, 1H)..
APCI-MS: m/z 434 (MH⁺).

### (iv) N-[2-({1-[4-(4-Fluorophenyl)-4-oxobutyl)-3-piperidinyl}methoxy)phenyl]-acetamide

A mixture of compound {1-[4-(4-fluorophenyl)-4oxobutyl]-3-piperidinyl}methyl benzenesulfonate obtained from step (iii) (137 mg, 0.268 mmol), 2-acetamidophenol (61 mg, 0.402 mmol), potassium carbonate (K₂CO₃) (350 mg, 2.53 mmol) in DMF(3 mL) was kept at 65°C for 4h. The reaction mixture was partitioned between ethylacetate and water (H₂O), organic layer was dried over sodium sulphate (Na₂SO₄), filtered and concentrated. The residue was purified by silica gel flash chromatography and converted to the corresponding hydrochloride salt to give the titled compound (72 mg).

¹H-NMR (CDCl₃, 400 MHz): δ 8.34 (d, J = 7.8 Hz, 1H); 8.00 (m, 3H); 7.10 (t, J = 8.7 Hz, 2H); 6.98 (m, 2H); 6.80 (d, J = 7.9 Hz, 1H); 3.85 (m, 2H); 3.07 (m, 2H); 2.82 (m, 2H); 2.45 (t, J = 7.0 Hz, 2H); 2.20 (s, 3H); 2.08 (m, 3H); 1.92 (m, 1H); 1.82 (t, J = 10.4 Hz, 1H) 1.68 (m, 2H); 1.45 (m, 1H); 1.08 (m, 1H).
APCI-MS: m/z 413 (MH⁺).

The compounds of Examples 4 to 34 were prepared by processes similar to those described in the previous Examples.

### Example 4

### N-[2-({4-(4-Fluorophenyl)4-oxobutyl]-2-morpholinyl}methoxy)phenyl]acetamide

APCI-MS: m/z 415 (MH⁺).

### Example 5

### 1-(4-Fluorophenyl)-4-{2-[3-methoxyphenoxy)methyl]-4-morpholinyl}-1-butanone

APCI-MS: m/z 388 (MH⁺).

### Example-6

### N-[2-({(3R)-1-[4-(4-Fluorophenyl)-4-oxobutyl]-3-piperidinyl}methoxy)phenyl]-acetamide

¹H-NMR (CDCl₃, 400 MHz): δ 8.35 (d, J = 7.6 Hz, 1H); 7.98 (m 3H); 7.14 (m, 2H); 7.17-6.95 (m, 2H); 6.84 (d, J = 8.0 Hz, 1H); 3.90 (m, 2H); 3.26 (br.s, 1H); 3.18-2.92 (m, 3H); 2.65 (br.s, 2H); 2.42-2.13 (m, 6H); 2.05 (m, 2H); 1.80 (m, 3H); 1.18 (m, 1H). ).
APCI-MS: m/z 413 (MH⁺).

### Example-7

### N-[2-({(3S)-1-[4-(4-Fluorophenyl)-4-oxobutyl]-3-piperidinyl}methoxy)phenyl]-acetamide

¹H-NMR (CDCl₃, 400 MHz): δ 8.35 (d, J = 7.6 Hz, 1H); 7.98 (m 3H); 7.14 (m, 2H); 7.17-6.95 (m, 2H); 6.84 (d, J = 8.0 Hz, 1H); 3.90 (m, 2H); 3.26 (br.s, 1H); 3.18-2.92 (m, 3H); 2.65 (br.s, 2H); 2.42-2.13 (m, 6H); 2.05 (m, 2H); 1.80 (m, 3H); 1.18 (m, 1H).).. APCI-MS: m/z 413 (MH⁺).

### Example-8

### N-[2-({1-[4-(4-Fluorophenyl)-4-oxobutyl]-3-piperidinyl}methoxy)phenyl]-benzamide

### (i) N-(2-Hydroxyphenyl)benzamide

To a mixture of 2-aminophenol (1.09 g, 10.0 mmol) and triethylamine (2.09 mL, 15.0 mmol) in THF (20 mL) was added benzoyl chloride (1.16 mL, 10.0 mmol) in THF (4 mL) dropwise over a period of 5 min at 0 °C. After addition was complete the reaction mixture was kept on stirring at room temperature for overnight. The reaction mixture was concentrated at reduced pressure. The residue was taken up in methanol, aqueous sodium hydroxide (NaOH) (8M, 5 mL) was added. After 5 min the pH of the reaction mixture was adjusted to 7.0 by addition of glacial acetic acid and concentrated in vacuo. The reaction mixture was dissolved in dichloromethane (CH₂Cl₂), washed successively with 1M aqueous hydrochloric acid (HCl), saturated aqueous sodium hydrogen carbonate (NaHCO₃). The organic layer was dried over sodium sulphate (Na₂SO₄) filtered and concentrated to give desired product (1.69 g).
¹H-NMR (DMSO-d₆, 400MHz): δ, 9.80 (s, 1H); 9.58 (s, 1H); 8.00 (m, 2H); 7.70 (m, 1H); 7.60 (m, 1H); 7.58 (m, 3H); 7.08 (m, 1H); 6.90 (m, 1H); 6.84 (m, 1H).

### (ii) N-[2-({1-[4-(4-Fluorophenyl)-4-oxobutyl]-3-piperidinyl}methoxy)phenyl] benzamide

APCI-MS: m/z 475 (MH⁺).

### Example-9

### 4-{3-[(2-Acetylphenoxy)methyl]-1-piperidinyl}-1-(4-fluorophenyl)-1-butanone

APCI-MS: m/z 398 (MH⁺).

### Example-10

### 4-{3-[(3-Benzoylphenoxy)methyl]-1-piperidinyl}-1-(4-fluorophenyl)-1-butanone

APCI-MS: m/z 460 (MH⁺).

### Example-11

### 4-{3-[(1,3-Benzothiazol-2-yloxy)methyl]-1-piperidinyl}-1-(4-fluorophenyl)-1-butanone

APCI-MS: m/z 413 (MH⁺).

### Example-12

### 1-(4-Fluorophenyl)-4-{3-[(8-quionolinyloxy)methyl]-1-piperidinyl}-1-butanone

APCI-MS: m/z 407 (MH⁺).

### Example-13

### 1-(4-Fluorophenyl)-4-(3-{[(2-methyl-8-quinolinyl)oxy]methyl}-1-piperidinyl)-1-butanone

APCI-MS: m/z 421 (MH⁺).

### Example-14

### 1-(4-Fluorophenyl)-4-{3-[(4-quinozolinyloxy)methyl]-1-piperidinyl}-1-butanone

APCI-MS: m/z 408 (MH⁺).

### Example-15

### 1-(4-Fluorophenyl)-4-{3-[(1-naphthyloxy)methyl]-1-piperidinyl}-1-butanone

APCI-MS: m/z 413 (MH⁺).

### Example-16

### 1-(4-Fluorophenyl)-4-(3-{[2-(5-isoxazolyl)phenoxy]methyl}-1-piperidinyl)-1-butanone

APCI-MS: m/z 423 (MH⁺).

### Example-17

### 4-(3-{[4-Chloro-2-(1H-pyrazol-5-yl)phenoxy]methyl}-1-piperidinyl)-1-(4-fluorophenyl)-1-butanone

APCI-MS: m/z 456 (MH⁺).

### Example-18

### N-Cyclopropyl-2-({1-[4-(4-fluorophenyl)-4-oxobutyl]-3-piperidinyl}methoxy)-benzamide

### (i) N-Cyclopropyl-2-hydroxybenzamide

A mixture ofmethylsalicylate (4.36 g, 28.69 mmol) and cyclopropylamine (1.64 g, 28.69 mmol) was heated at 80-100 °C for 3h. An additional 0.5 equivalent of cyclopropylamine was added and the reaction mixture was kept at 70 °C for overnight. The reaction mixture was co-evaporated with toluene and the residue was purified by silica gel flash chromatography to give the subtitle compound (2.71 g).

¹H-NMR (CDCl₃, 400 MHz): δ 12.36 (s, 1H); 7.40 (m, 1H); 7.31 (m, 1H); 7.00 (dd, J = 0.9 Hz, 8.4 Hz, 1H); 6.83 (m, 1H); 6.53 (br.s, 1H); 2.89 (m, 1H); 0.93 (m, 2H); 0.67 (m, 2H).

### (ii) N-Cyclopropyl-2-({1-[4-(4-fluorophenyl)-4-oxobutyl]-3-piperidinyl}methoxy)-benzamide

APCI-MS: m/z 439 (MH⁺).

### Example-19

### Methyl 2-({1-[4-(4-fluorophenyl)-4-oxobutyl]-3-piperidinyl}methoxy)benzoate

APCI-MS: m/z 414 (MH⁺).

### Example-20

### 2-({1-[4-(4-Fluorophenyl)-4-oxobutyl]-3-piperidinyl}methoxy)-N-methylbenzamide

### (i) 3-Hydroxy-N-methylbenzamide

A mixture of 3-hydroxybenzoic acid (1.3 g, 9.4 mmol) and a ethanolic methylamine solution (33 %, 1.5 ml, 12.1 mmol) were stirred at 60°C for 48 h, then the solvent was evaporated in vacuo, and the residue redissolved in a small volume of ethanol. The product precipitated from the solution by the addition of ethyl acetate. The precipitate was collected by filtration and dried to give the subtitle compound (1.3 g, 91 %).
¹H-NMR (400MHz, DMSO-d₆): δ 7.32 (m, 1H), 7.29 (d, 1H, *J* = *7.6*), 7.09 (t, 1H, *J* = *7.6*), 6.73 (dm, 1H, *J* = *7.6*), 2.37 (s, 3H).

### (ii) 2-({1-[4-(4-Fluorophenyl)-4-oxobutyl]-3-piperidinyl}methoxy)-N-methylbenzamide

APCI-MS: m/z 413 (MH⁺).

### Example-21

### 1-(4-Fluorophenyl)-4-[3-({2-[(E)-1-propenyl]phenoxy}methyl)-1-piperidinyl]-1-butanone

APCI-MS: m/z 396 (MH⁺).

### Example-22

### 4-{3-[3,5-Difluorophenoxy)methyl]-1-piperidinyl}-1-(4-fluorophenyl)-1-butanone

APCI-MS: m/z 392 (MH⁺).

### Example-23

### 4-[3-[(4-Chloro-2-isopropyl-5-methylphenoxy)methyl]-1-piperidinyl}-1-(4-fluorophenyl)-1-butanone

APCI-MS: m/z 446 (MH⁺).

### Example-24

### 4-{3-[3,4-Dinitrophenoxy)methyl]-1-piperidinyl}-1-(4-fluorophenyl)-1-butanone

APCI-MS: m/z 446 (MH⁺).

### Example-25

### 1-(4-Fluorophenyl)-4-{3-[(2-isopropoxyphenoxy)methyl]-1-piperidinyl}-1-butanone

APCI-MS: m/z 414 (MH⁺).

### Example-26

### 1-(4-Fluorophenyl)-4-{3-[(2-propionylphenoxy)methyl]-1-piperidinyl}-1-butanone

APCI-MS: m/z 412(MH⁺).

### Example-27

### 4-{3-[(2-Butyrylphenoxy)methyl]-1-piperidinyl}-1-(4-fluorophenyl)-1-butanone

APCI-MS: m/z 426 (MH⁺).

### Example-28

### 1-(4-Fluorophenyl)-4-(3-{[(3-methyl-1,2-benzisoxazol-4-yl)-oxy]methyl}-1-piperidinyl)-1-butanone

### (i) 2-Methyl-1,3-benzoxazol4-ol

A solution of 2,6-dihydroxyacetophenone (1.43 g, 9.4 mmol), hydroxylamine hydrochloride (940 mg, 13.6 mmol, potassium hydroxide (KOH) (1.15 g, 20.6 mmol), and water (10 mL) in methanol (15 mL) was heated at reflux temperature under nitrogen for 18 h. The reaction mixture was cooled to room temperature and concentrated in vacuo. The residue was partitioned between ethylacetate and water. The organic layer was extracted with 1M hydrochloric acid (HCl), dried over sodium sulphate (Na₂SO₄), filtered and concentrated. The residue was purified by silica gel flash chromatography to give the subtitle compound (673 mg).
¹H-NMR (acetone-d₆ 400 MHz): δ 9.55 (br.s, 1H); 7.36 (m, 1H); 7.02 (d, J = 8.3 Hz, 1H); 6.68 (d, J = 7.8 Hz, 1H); 2.60 (s, 3H).
APCI-MS: m/z 150 (MH⁺).

### (ii) 1-(4-Fluorophenyl)-4-(3-{[(3-methyl-1,2-benzisoxazol-4-yl)-oxy]methyl}-1-piperidinyl)-1-butanone

APCI-MS: m/z 411 (MH⁺).

### Example-29

### N-[2-({1-[4-(4-Fluorophenyl)-4-oxobutyl]-3-piperidinyl}methoxy)phenyl]urea

APCI-MS: m/z 413 (MH⁺).

### Example-30

### 5-Chloro-2-({1-[4-(4-fluorophenyl)-4-oxobutyl]-3-piperidinyl}methoxy)benzamide

APCI-MS: m/z 433 (MH⁺).

### Example-31

### N-Ethyl-N'-[2-({1-[4-(4-fluorophenyl)-4-oxobutyl]-3-piperidinyl}methoxy)-phenyl]urea

### (i) N-Ethyl-N'-(2-hydroxyphenyl)urea

A solution of 1-isocyanato-2-methoxybenzene (0.32 g, 2.15 mmol) and ethylamine (1 mL) in dichloromethane (15 mL) was stirred at room temperature for 2 days. Then the volatiles were removed in vacuo. The residue was redissolved in dichloromethane (15 mL), and boron tribromide (BBr₃) (1 M in dichloromethane (CH₂Cl₂), 6.5 ml, 6.5 mmol) was added dropwise via syringe under nitrogen. After stirring for 1 h the reaction mixture was diluted with dichloromethane and washed 3 times with water. The product was purified by HPLC (Kromasil column; eluent: acetonitrile + 0.1 % trifluoroacetic acid (TFA)/water + 0.1 % TFA) to give the subtitle compound (167 mg).

¹H-NMR (400 MHz, DMSO-d₆): δ 7.90 (br. s, 1H), 7.82 (dd, 1H, *J* = *10.0, J* = *2.4*).), 6.6 - 6.9 (m, 3H), 3.01 (quart, 2H, ³*J* = *9.6*) 1.05 (t, 3H, ³*J* = *9.6*).
APCI-MS: m/z 181 (MH⁺).

### (ii) N-Ethyl-N'-[2-({1-[4-(4-fluorophenyl)-4-oxobutyl]-3-piperidinyl}methoxy)-phenyl]urea

APCl-MS: m/z 442 (MH⁺).

### Example-32

### N'-[2-({1-[4-(4-Fluorophenyl)-4-oxobutyl]-3-piperidinyl}methoxy)phenyl]-N,N-dimethylurea

### (i) N'-(2-hydroxyphenyl)-N,N-dimethylurea

The subtitled compound was prepared using the procedure as described for Example 31 for N-ethyl-N'-(2-hydroxyphenyl)urea from 1-isocyanato-2-methoxybenzene and dimethylamine (2M solution in THF). Yield 54%.

¹H-NMR (400 MHz, DMSO-d₆): δ 7.78 (br. s, 1H), 7.49 (dd, 1H, *J* = *10.4, J* = *2.4*).), 6.7 - 7.0 (m, 3H), 2.97 (s, 6H).
APCI-MS: m/z 181 (MH⁺).

### (ii) N'-[2-({1-[4-(4-Fluorophenyl)-4-oxobutyl]-3-piperidinyl}methoxy)phenyl]-N,N-dimethylurea

APCI-MS: m/z 442 (MH⁺).

### Example-33

### 4-(3-{[2-(Benzylamino)phenoxy]methyl}-1-piperidinyl)-1-(4-fluorophenyl)-1-butanone

### (i) 2-(Benzylamino)phenol

To a stirred mixture of 2-aminophenol (3.0 g, 27.5 mmol), potassium carbonate (6.0 g, 43.4 mmol) and DMF (25 mL) 1-(bromomethyl)benzene (3.75 mL, 31.3 mmol) was added. The mixture was stirred at 60 ^{°C} for overnight. Purification by preparative HPLC (Kromasil C₁₈; eluent: acetonitrile + 0.1 % TFA/water + 0.1 % TFA) gave the subtitled compound (1.82 g, 33 %).

¹H-NMR (400 MHz, DMSO-d₆): δ 7.2-7.4 (m, 5H), 6.6-6.9 (m, 4H), 4.37 (s, 2H). APCI-MS: m/z 200 (MH⁺).

### (ii) 4-(3-{[2-(Benzylamino)phenoxy]methyl}-1-piperidinyl)-1-(4-fluorophenyl)-1-butanone

APCI-MS: m/z 461 (MH⁺).

### Example-34

### 1-(4-Fluorophenyl)-4-(3-{[(2-methyl-1,3-benzoxazol-4-yl)oxy]methyl}-1-piperidinyl)-1-butanone

### (i) N-(2,6-dihydroxyphenyl)acetamide:

A mixture of 2-nitro-1,3-benzenediol (1.55 g 10 mmol), acetic anhydride (1.59 g, 1.47 mL, 15 mmol) and 10 % palladium on charcoal (0.3 g) in methanol (100 mL) was stirred in the atmosphere of hydrogen at atmospheric pressure for 2 h. The catalyst was filtered through celite, the solvent evaporated in vacuo. The oily residue was treated with dichloromethane to afford colourless crystals, which were collected by filteration and dried to give the subtitled compound (1.09 g, 65 %).

¹H-NMR (400 MHz, DMSO-d₆): δ 9.34 (br. s, 1H), 6.87 (t, 1H, *J* = *8. 0*), 6.34 (d, 2H, *J* = *8.0*), 2.10 (s, 3H).
APCI-MS: m/z 168 (MH⁺).

### (ii) 2-Methyl-1,3-benzoxazol-4-ol

*N*-(2,6-dihydroxyphenyl)acetamide was heated at 200 °C for 0.5 h. After cooling to room temperature, the product was purified by flash chromatography on silica gel (ethyl acetate/heptane, 1 : 1) to afford the subtitled compound as colourless crystals (0.77 g, 79%).

¹H-NMR (400 MHz, DMSO-d₆): δ 10.15 (br. s, 1H), 7.11 (t, 1H, *J* = *8. 0*), 7.04 (d, 1H, *J* = *8.0*), 6.70 (d, 1H, *J* = *8.0*), 2.56 (s, 3H).
APCI-MS: m/z 150 (MH⁺)

### (iii) 1-(4-Fluorophenyl)-4-(3-{[(2-methyl-1,3-benzoxazol-4-yl)oxy]methyl}-1-piperidinyl)-1-butanone

APCI-MS: m/z 411 (MH⁺).

### THP-1 Chemotaxis Assay

### Introduction

The assay measured the chemotactic response elicited by MIP-1α, chemokine in the human monocytic cell line THP-1. The compounds of the Examples were evaluated by their ability to depress the chemotactic response to a standard concentration of MIP-1α chemokine.

### Methods

### Culture of THP-1 cells

Cells were thawed rapidly at 37°C from frozen aliquots and resuspended in a 25 cm flask containing 5 ml of RPMI-1640 medium supplemented with Glutamax and 10% heat inactivated fetal calf serum without antibiotics (RPMI+10%HIFCS). At day 3 the medium is discarded and replaced with fresh medium.

THP-1 cells are routinely cultured in RPMI-1640 medium supplemented with 10% heat inactivated fetal calf serum and glutamax but without antibiotics. Optimal growth of the cells requires that they are passaged every 3 days and that the minimum subculture density is 4x10+5 cells/ml.

### Chemotaxis assay

Cells were removed from the flask and washed by centrifugation in RPMI+10%HIFCS+glutamax. The cells were then resuspended at 2x10+7 cells/ml in fresh medium (RPMI+10%HIFCS+glutamax) to which was added calcein-AM (5 µl of stock solution to 1 ml to give a final concentration of 5x10⁻⁶M). After gentle mixing the cells were incubated at 37°C in a CO₂ incubator for 30 minutes. The cells were then diluted to 50 ml with medium and washed twice by centrifugation at 400xg. Labelled cells were then resuspended at a cell concentration of 1x10+7 cells/ml and incubated with an equal volume of MIP-1α antagonist (10⁻¹⁰M to 10⁻⁶M final concentration) for 30 minutes at 37°C in a humidified CO₂ incubator.

Chemotaxis was performed using Neuroprobe 96-well chemotaxis plates employing 8 µm filters (cat no. 101-8). Thirty microlitres of chemoattractant supplemented with various concentrations of antagonists or vehicle were added to the lower wells of the plate in triplicate. The filter was then carefully positioned on top and then 25µl of cells preincubated with the corresponding concentration of antagonist or vehicle were added to the surface of the filter. The plate was then incubated for 2 hours at 37°C in a humidified CO₂ incubator. The cells remaining on the surface were then removed by adsorption and the whole plate was centrifuged at 2000 rpm for 10 minutes. The filter was then removed and the cells that had migrated to the lower wells were quantified by the fluorescence of cell associated calcein-AM. Cell migration was then expressed in fluorescence units after subtraction of the reagent blank and values were standardized to % migration by comparing the fluorescence values with that of a known number of labelled cells. The effect of antagonists was calculated as % inhibition when the number of migrated cells were compared with vehicle.

## Claims

1. A compound of formula 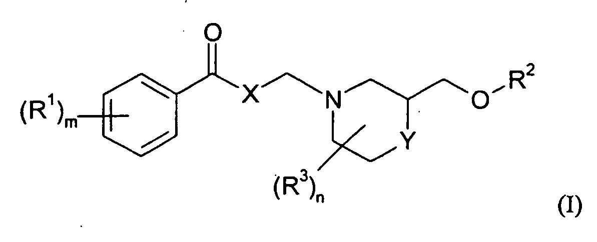 wherein
m is 0, 1, 2 or 3;
each R¹ independently represents halogen, cyano, nitro, carboxyl, hydroxyl, C₃-C₆ cycloalkyl, C₁-C₆ alkoxy, C₁-C₆ alkoxycarbonyl, C₁-C₆ haloalkyl, C₁-C₆ haloalkoxy, -NR⁶R⁷, C₃-C₆ cycloalkylamino, C₁-C₆ alkylthio, C₁-C₆ alkylcarbonyl, C₁-C₆ alkylcarbonylamino, sulphonamido, C₁-C₆ alkylsulphonyl, -C(O)NR⁸R⁹, -NR¹⁰C(O)-(NH)ₚR¹¹, phenyl, or C₁-C₆ alkyl optionally substituted by at least one substituent selected from carboxyl and C₁-C₆ alkoxycarbonyl;
X represents a bond or a group CH₂ or (CH₂)₂;
Y represents an oxygen atom or a group CH₂;
R² represents an unsaturated 5- to 10-membered ring system which may comprise at least one ring heteroatom selected from nitrogen, oxygen and sulphur, the ring system being optionally substituted by at least one substituent selected from halogen, cyano, nitro, carboxyl, hydroxyl, C₂-C₆ alkenyl, C₃-C₅ cycloalkyl, C₁-C₆ alkoxy, C₁-C₆ alkoxycarbonyl, C₁-C₆ haloalkyl, C₁-C₆ haloalkoxy, -NR¹²R¹³, C₃-C₆ cycloalkylamino, C₁-C₆ alkylthio, C₁-C₆ alkylcarbonyl, phenylcarbonyl, C₁-C₆ alkylcarbonylamino, sulphonamido, C₁-C₆ alkylsulphonyl, -C(O)NR¹⁴R¹⁵, C₁-C₆ alkoxycarbonylC₁-C₆ alkyl, phenyl, isoxazolyl, pyrazolyl, -NHSO₂CH₃, -NHC(O)NR¹⁶R¹⁷, -OC(O)NR¹⁸R¹⁹, -OCH₂C(O)NR²⁰R²¹, -NHC(O)OR²², -NHC(O)R²³, and C₁-C₆ alkyl itself optionally substituted by at least one substituent selected from carboxyl and C₁-C₆ alkoxycarbonyl;
n is 0, 1 or 2;
each R³ independently represents a C₁-C₆ alkyl, C₁-C₆ alkoxycarbonyl, -CH₂OH or carboxyl group;
R⁶ and R⁷ each independently represent a hydrogen atom or a C₁-C₆ alkyl group, or R⁶ and R⁷ together with the nitrogen atom to which they are attached form a 4- to 7-membered saturated heterocycle;
R⁸ and R⁹ each independently represent a hydrogen atom or a C₁-C₆ alkyl group optionally substituted by at least one C₁-C₆ alkoxycarbonyl;
p is 0 or 1;
R¹⁰ represents a hydrogen atom or a C₁-C₆ alkyl group;
R¹¹ represents a hydrogen atom, or a C₁-C₆ alkyl group optionally substituted by at least one substituent selected from carboxyl, C₁-C₆ alkoxy and C₁-C₆ alkoxycarbonyl;
R¹² and R¹³ each independently represent a hydrogen atom, or a C₁-C₆ alkyl group optionally substituted by at least one substituent selected from phenyl, carboxyl and C₁-C₆ alkoxycarbonyl, or R¹² and R¹³ together with the nitrogen atom to which they are attached form a 4- to 7-membered saturated heterocycle;
R¹⁴ and R¹⁵ each independently represent a hydrogen atom, a C₃-C₆ cycloalkyl group, or a C₁-C₆ alkyl group optionally substituted by at least one substituent selected from carboxyl and C₁-C₆ alkoxycarbonyl, or R¹⁴ and R¹⁵ together with the nitrogen atom to which they are attached form a 4- to 7-membered saturated heterocycle;
R¹⁶ and R¹⁷ each independently represent a hydrogen atom, or a C₁-C₆ alkyl group optionally substituted by at least one substituent selected from carboxyl and C₁-C₆ alkoxycarbonyl, or R¹⁶ and R¹⁷ together with the nitrogen atom to which they are attached form a 4- to 7-membered saturated heterocycle;
R¹⁸ and R¹⁹ each independently represent a hydrogen atom, or a C₁-C₆ alkyl group optionally substituted by at least one substituent selected from carboxyl and C₁-C₆ alkoxycarbonyl, or R¹⁸ and R¹⁹ together with the nitrogen atom to which they are attached form a 4- to 7-membered saturated heterocycle;
R²⁰ and R²¹ each independently represent a hydrogen atom, or a C₁-C₆ alkyl group optionally substituted by at least one substituent selected from carboxyl and C₁-C₆ alkoxycarbonyl, or R²⁰ and R²¹ together with the nitrogen atom to which they are attached form a 4- to 7-membered saturated heterocycle;
R²² represents a hydrogen atom, or a C₁-C₆ alkyl group optionally substituted by at least one substituent selected from carboxyl and C₁-C₆ alkoxycarbonyl;
R²³ represents a group C₁-C₆ alkyl, C₂-C₆ alkenyl, C₃-C₆ cycloalkyl, adamantyl, C₅-C₆ cycloalkenyl, phenyl or a saturated or unsaturated 5- to 10-membered heterocyclic ring system comprising at least one ring heteroatom selected from nitrogen, oxygen and sulphur, each group being optionally substituted by at least one substituent selected from nitro, hydroxyl, oxo, halogen, carboxyl, C₁-C₆ alkyl, C₁-C₆ alkoxy, C₁-C₆ alkylthio, C₁-C₆ alkylcarbonyl, C₁-C₆ alkoxycarbonyl, phenyl and -NHC(O)-R²⁴; and
R²⁴ represents a C₁-C₆ alkyl, amino (-NH₂) or phenyl group;
or a pharmaceutically acceptable salt or solvate thereof.

2. A compound according to claim 1, wherein each R¹ independently represents halogen, cyano, nitro, C₁-C₆ alkoxy, C₁-C₆ alkylcarbonyl or C₁-C₆ alkylcarbonylamino.

3. A compound according to claim 1 or claim 2, wherein X represents a group CH₂ or (CH₂)₂.

4. A compound according to any one of claims 1 to 3, wherein Y represents a group CH₂.

5. A compound according to any one of claims 1 to 4, wherein the unsaturated 5- to 10-membered ring system in R² is selected from phenyl, naphthyl, 1,3-benzodioxolyl, pyrazolyl, thienyl, oxazolyl, imidazolyl, pyridinyl, pyridopyrrolyl, benzimidazolyl, indazolyl, benzothiazolyl, quinolinyl, isoquinolinyl, quinazolinyl, quinoxalinyl, benzisoxazolyl, benzoxazolyl, thiazolyl and benzotriazolyl.

6. A compound according to any one of claims 1 to 5, wherein the unsaturated 5- to 10-membered ring system in R² is optionally substituted by at least one substituent selected from halogen, nitro, C₂-C₆ alkenyl, C₁-C₆ alkoxy, C₁-C₆ alkoxycarbonyl, C₁-C₆ alkylcarbonyl, phenylcarbonyl, C₁-C₆ alkyl, isoxazolyl, pyrazolyl, -NR¹²R¹³, ₋C(O)NR¹⁴R¹⁵, -NHC(O)NR¹⁶R¹⁷, and -NHC(O)R²³_{.}

7. A compound according to claim 1 being selected from:
N-[2-({4-(4- fluorophenyl)-4-oxobutyl]-2-morpholinyl}methoxy)phenyl]acetamide,
N-[2-({3-(4-Fluorophenyl)-3-oxopropyl]-2-morpholinyl}methoxy)phenyl]acetamide,
N-[2-({1-[4-(4-Fluorophenyl)-4-oxobutyl)-3-piperidinyl}methoxy)phenyl]acetamide,
N-[2-({4-(4-Fluorophenyl)-4-oxobutyl]-2-morpholinyl}methoxy)phenyl]acetamide,
1-(4-Fluorophenyl)-4-{2-[3-methoxyphenoxy)methyl]-4-morpholinyl}-1-butanone,
N-[2-({(3R)-1-[4-(4-Fluorophenyl)-4-oxobutyl]-3-piperidinyl}methoxy)phenyl]-acetamide,
N-[2-({(3S)-1-[4-(4-Fluorophenyl)-4-oxobutyl]-3-piperidinyl}methoxy)phenyl]-acetamide,
N-[2-({1-[4-(4-Fluorophenyl)-4-oxobutyl]-3-piperidinyl}methoxy)phenyl]-benzamide,
4-{3-[(2-Acetylphenoxy)methyl]-1-piperidinyl}-1-(4-fluorophenyl)-1-butanone,
4-{3-[(3-Benzoylphenoxy)methyl]-1-piperidinyl}-1-(4-fluorophenyl)-1-butanone,
4-{3-[(1,3-Benzothiazol-2-yloxy)methyl]-1-piperidinyl}-1-(4-fluorophenyl)-1-butanone,
1-(4-Fluorophenyl)-4-{3-[(8-quionolinyloxy)methyl]-1-piperidinyl}-1-butanone,
1-(4-Fluorophenyl)-4-(3-{[(2-methyl-8-quinolinyl)oxy]methyl}-1-piperidinyl)-1-butanone,
1-(4-Fluorophenyl)-4-{3-[(4-quinozolinyloxy)methyl]-1-piperidinyl}-1-butanone,
1-(4-Fluorophenyl)-4-{3-[(1-naphthyloxy)methyl]-1-piperidinyl}-1-butanone,
1-(4-Fluorophenyl)-4-(3-{[2-(5-isoxazolyl)phenoxy]methyl}-1-piperidinyl)-1-butanone,
4-(3-{[4-Chloro-2-(1H-pyrazol-5-yl)phenoxy]methyl}-1-piperidinyl)-1-(4-fluorophenyl)-1-butanone,
N-Cyclopropyl-2-({1-[4-(4-fluorophenyl)-4-oxobutyl]-3-piperidinyl}methoxy)-benzamide,
Methyl 2-({1-[4-(4-fluorophenyl)-4-oxobutyl]-3-piperidinyl}methoxy)benzoate,
2-({1-[4-(4-Fluorophenyl)-4-oxobutyl]-3-piperidinyl}methoxy)-N-methylbenzamide,
1-(4-Fluorophenyl)-4-[3-({2-[(E)-1-propenyl]phenoxy}methyl)-1-piperidinyl]-1-butanone,
4-{3-[3,5-Difluorophenoxy)methyl]-1-piperidinyl}-1-(4-fluorophenyl)-1-butanone,
4-[3-[(4-Chloro-2-isopropyl-5-methylphenoxy)methyl]-1-piperidinyl}-1-(4-fluorophenyl)-1-butanone,
4-{3-[3,4-Dinitrophenoxy)methyl]-1-piperidinyl}-1-(4-fluorophenyl)-1-butanone,
1-(4-Fluorophenyl)-4-{3-[(2-isopropoxyphenoxy)methyl]-1-piperidinyl}-1-butanone,
1-(4-Fluorophenyl)-4-{3-[(2-propionylphenoxy)methyl]-1-piperidinyl}-1-butanone,
4-{3-[(2-Butyrylphenoxy)methyl]-1-piperidinyl}-1-(4-fluorophenyl)-1-butanone,
1-(4-Fluorophenyl)-4-(3-{[(3-methyl-1,2-benzisoxazol-4-yl)-oxy]methyl}-1-piperidinyl)-1-butanone,
N-[2-({1-[4-(4-Fluorophenyl)-4-oxobutyl]-3-piperidinyl}methoxy)phenyl]urea,
5-Chloro-2-({1-[4-(4-fluorophenyl)-4-oxobutyl]-3-piperidinyl}methoxy)benzamide,
N-Ethyl-N'-[2-({1-[4-(4-fluorophenyl)-4-oxobutyl]-3-piperidinyl}methoxy)-phenyl]urea,
N'-[2-({1-[4-(4-Fluorophenyl)-4-oxobutyl]-3-piperidinyl}methoxy)phenyl]-N,N-dimethylurea,
4-(3-{[2-(Benzylamino)phenoxy]methyl}-1-piperidinyl)-1-(4-fluorophenyl)-1-butanone,
1-(4-Fluorophenyl)-4-(3-{[(2-methyl-1,3-benzoxazol-4-yl)oxy]methyl}-1-piperidinyl)-1-butanone,
and pharmaceutically acceptable salts and solvates of any one thereof.

8. A process for the preparation of a compound of formula (I) as defined in claim 1 which comprises reacting a compound of formula 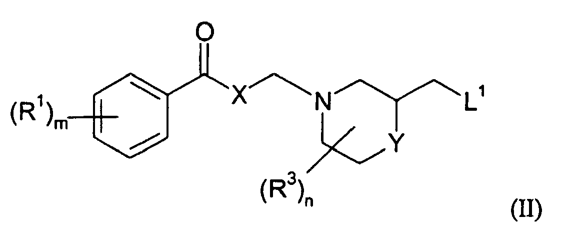 wherein L¹ represents a leaving group and m, n, X, Y, R¹ and R³ are as defined in formula (I), with a compound of formula
R²-OH (II)
wherein R² is as defined in formula (I);
and optionally thereafter forming a pharmaceutically acceptable salt or solvate of the compound of formula (I) obtained.

9. A pharmaceutical composition comprising a compound of formula (I), or a pharmaceutically acceptable salt or solvate thereof, as claimed in any one of claims 1 to 7 in association with a pharmaceutically acceptable adjuvant, diluent or carrier.

10. A process for the preparation of a pharmaceutical composition as claimed in claim 9 which comprises mixing a compound of formula (I), or a pharmaceutically acceptable salt or solvate thereof, as claimed in any one of claims 1 to 7 with a pharmaceutically acceptable adjuvant, diluent or carrier.

11. A compound of formula (I), or a pharmaceutically-acceptable salt or solvate thereof, as claimed in any one of claims 1 to 7 for use in therapy.

12. Use of a compound of formula (I), or a pharmaceutically acceptable salt or solvate thereof, as claimed in any one of claims 1 to 7 in the manufacture of a medicament for the treatment of human diseases or conditions in which modulation of chemokine receptor activity is beneficial.

13. Use of a compound of formula (I), or a pharmaceutically acceptable salt or solvate thereof, as claimed in any one of claims 1 to 7 in the manufacture of a medicament for use in treating rheumatoid arthritis.

14. Use of a compound of formula (I), or a pharmaceutically acceptable salt or solvate thereof, as claimed in any one of claims 1 to 7 in the manufacture of a medicament for use in treating chronic obstructive pulmonary disease.

15. Use of a compound of formula (I), or a pharmaceutically acceptable salt or solvate thereof, as claimed in any one of claims 1 to 7 in the manufacture of a medicament for use in treating asthma.

16. Use of a compound of formula (I), or a pharmaceutically acceptable salt or solvate thereof, as claimed in any one of claims 1 to 7 in the manufacture of a medicament for use in treating multiple sclerosis.

17. Use of a compound of formula (I), or a pharmaceutically acceptable salt or solvate thereof, as claimed in any one of claims 1 to 7 in the manufacture of a medicament for use in treating an inflammatory disease.

18. Use of a compound of formula (I), or a pharmaceutically acceptable salt or solvate thereof, as claimed in any one of claims 1 to 7 in the manufacture of a medicament for use in treating an airways disease.

## Patentansprüche

1. Verbindung der Formel 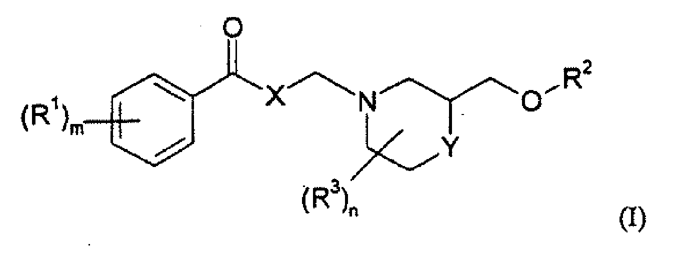 worin
m für 0, 1, 2 oder 3 steht;
R¹ jeweils unabhängig voneinander für Halogen, Cyano, Nitro, Carboxyl, Hydroxyl, C₃-C₆-Cycloalkyl, C₁-C₆-Alkoxy, C₁-C₆-Alkoxycarbonyl, C₁-C₆-Halogenalkyl, C₁-C₆-Halogenalkoxy, -NR⁶R⁷, C₃-C₆-Cycloalkylamino, C₁-C₆-Alkylthio, C₁-C₆-Alkylcarbonyl, C₁-C₆-Alkylcarbonylamino, Sulfonamido, C₁-C₆-Alkylsulfonyl, -C(O)NR⁸R⁹, -NR¹⁰C(O)-(NH)ₚR¹¹, Phenyl oder gegebenenfalls durch mindestens einen unter Carboxyl und C₁-C₆-Alkoxycarbonyl ausgewählten Substituenten substituiertes C₁-C₆-Alkyl steht; X für eine Bindung oder eine Gruppe CH₂ oder (CH₂)₂ steht;
Y für ein Sauerstoffatom oder eine Gruppe CH₂ steht;
R² für ein ungesättigtes 5- bis 10-gliedriges Ringsystem steht, das mindestens ein unter Stickstoff, Sauerstoff und Schwefel ausgewähltes Ringheteroatom enthalten kann und gegebenenfalls durch mindestens einen unter Halogen, Cyano, Nitro, Carboxyl, Hydroxyl, C₂-C₆-Alkenyl, C₃-C₅-Cycloalkyl, C₁-C₆-Alkoxy, C₁-C₆-Alkoxycarbonyl, C₁-C₆-Halogenalkyl, C₁-C₆-Halogenalkoxy, -NR¹²R¹³, C₃-C₆-Cycloalkylamino, C₁-C₆-Alkylthio, C₁-C₆-Alkylcarbonyl, Phenylcarbonyl, C₁-C₆-Alkylcarbonylamino, Sulfonamido, C₁-C₆-Alkylsulfonyl, -C(O)NR¹⁴R¹⁵, C₁-C₆-Alkoxycarbonyl-C₁-C₆-alkyl, Phenyl, Isoxazolyl, Pyrazolyl, -NHSO₂CH₃, -NHC(O)NR¹⁶R¹⁷, -OC(O)NR¹⁸R¹⁹, -OCH₂C(O)NR²⁰R²¹, -NHC(O)OR²², -NHC(O)R²³ und selbst gegebenenfalls durch mindestens einen unter Carboxyl und C₁-C₆-Alkoxycarbonyl ausgewählten Substituenten substituiertes C₁-C₆-Alkyl ausgewählten Substituenten substituiert ist;
n für 0, 1 oder 2 steht;
R³ jeweils unabhängig voneinander für eine C₁-C₆-Alkyl-, C₁-C₆-Alkoxycarbonyl-, -CH₂OH- oder Carboxylgruppe steht;
R⁶ und R⁷ jeweils unabhängig voneinander für ein Wasserstoffatom oder eine C₁-C₆-Alkylgruppe stehen oder gemeinsam mit dem Stickstoffatom, an das sie gebunden sind, einen 4- bis 7-gliedrigen gesättigten Heterocyclus bilden;
R⁸ und R⁹ jeweils unabhängig voneinander für ein Wasserstoffatom oder eine gegebenenfalls durch mindestens eine C₁-C₆-Alkoxycarbonylgruppe substituierte C₁-C₆-Alkylgruppe stehen;
p für 0 oder 1 steht;
R¹⁰ für ein Wasserstoffatom oder eine C₁-C₆-Alkylgruppe steht;
R¹¹ für ein Wasserstoffatom oder eine gegebenenfalls durch mindestens einen unter Carboxyl, C₁-C₆-Alkoxy und C₁-C₆-Alkoxycarbonyl ausgewählten Substituenten substituierte C₁-C₆-Alkylgruppe steht;
R¹² und R¹³ jeweils unabhängig voneinander für ein Wasserstoffatom oder eine gegebenenfalls durch mindestens einen unter Phenyl, Carboxyl und C₁-C₆-Alkoxycarbonyl ausgewählten Substituenten substituierte C₁-C₆-Alkylgruppe stehen oder gemeinsam mit dem Stickstoffatom, an das sie gebunden sind, einen 4- bis 7-gliedrigen gesättigten Heterocyclus bilden;
R¹⁴ und R¹⁵ jeweils unabhängig voneinander für ein Wasserstoffatom, eine C₃-C₆-Cycloalkylgruppe oder eine gegebenenfalls durch mindestens einen unter Carboxyl und C₁-C₆-Alkoxycarbonyl ausgewählten Substituenten substituierte C₁-C₆-Alkylgruppe stehen oder gemeinsam mit dem Stickstoffatom, an das sie gebunden sind, einen 4- bis 7-gliedrigen gesättigten Heterocyclus bilden;
R¹⁶ und R¹⁷ jeweils unabhängig voneinander für ein Wasserstoffatom oder eine gegebenenfalls durch mindestens einen unter Carboxyl und C₁-C₆-Alkoxycarbonyl ausgewählten Substituenten substituierte C₁-C₆-Alkylgruppe stehen oder gemeinsam mit dem Stickstoffatom, an das sie gebunden sind, einen 4-bis 7-gliedrigen gesättigten Heterocyclus bilden;
R¹⁸ und R¹⁹ jeweils unabhängig voneinander für ein Wasserstoffatom oder eine gegebenenfalls durch mindestens einen unter Carboxyl und C₁-C₆-Alkoxycarbonyl ausgewählten Substituenten substituierte C₁-C₆-Alkylgruppe stehen oder gemeinsam mit dem Stickstoffatom, an das sie gebunden sind, einen 4-bis 7-gliedrigen gesättigten Heterocyclus bilden;
R²⁰ und R²¹ jeweils unabhängig voneinander für ein Wasserstoffatom oder eine gegebenenfalls durch mindestens einen unter Carboxyl und C₁-C₆-Alkoxycarbonyl ausgewählten Substituenten substituierte C₁-C₆-Alkylgruppe stehen oder gemeinsam mit dem Stickstoffatom, an das sie gebunden sind, einen 4-bis 7-gliedrigen gesättigten Heterocyclus bilden;
R²² für ein Wasserstoffatom oder eine gegebenenfalls durch mindestens einen unter Carboxyl und
C₁-C₆-Alkoxycarbonyl ausgewählten Substituenten substituierte C₁-C₆-Alkylgruppe steht;
R²³ für eine C₁-C₆-Alkyl-, C₂-C₆-Alkenyl-, C₃-C₆-Cycloalkyl-, Adamantyl-, C₅-C₆-Cycloalkenyl- oder Phenylgruppe oder ein gesättigtes oder ungesättigtes 5- bis 10-gliedriges heterocyclisches Ringsystem, das mindestens ein unter Stickstoff, Sauerstoff und Schwefel ausgewähltes Ringheteroatom enthält, steht, wobei jede Gruppe gegebenenfalls durch mindestens einen unter Nitro, Hydroxyl, Oxo, Halogen, Carboxyl, C₁-C₆-Alkyl, C₁-C₆-Alkoxy, C₁-C₆-Alkylthio, C₁-C₆-Alkylcarbonyl, C₁-C₆-Alkoxycarbonyl, Phenyl und -NHC(O)-R²⁴ ausgewählten Substituenten substituiert ist; und R²⁴ für eine C₁-C₆-Alkyl-, Amino- (-NH₂-) oder Phenylgruppe steht;
oder ein pharmazeutisch annehmbares Salz oder Solvat davon.

2. Verbindung nach Anspruch 1, worin R¹ jeweils unabhängig voneinander für Halogen, Cyano, Nitro, C₁-C₆-Alkoxy, C₁-C₆-Alkylcarbonyl oder C₁-C₆-Alkylcarbonylamino steht.

3. Verbindung nach Anspruch 1 oder 2, worin X für eine Gruppe CH₂ oder (CH₂)₂ steht.

4. Verbindung nach einem der Ansprüche 1 bis 3, worin Y für eine Gruppe CH₂ steht.

5. Verbindung nach einem der Ansprüche 1 bis 4, worin das ungesättigte 5- bis 10-gliedrige Ringsystem in R² unter Phenyl, Naphthyl, 1,3-Benzodioxolyl, Pyrazolyl, Thienyl, Oxazolyl, Imidazolyl, Pyridinyl, Pyridopyrrolyl, Benzimidazolyl, Indazolyl, Benzothiazolyl, Chinolinyl, Isochinolinyl, Chinazolinyl, Chinoxalinyl, Benzisoxazolyl, Benzoxazolyl, Thiazolyl und Benzotriazolyl ausgewählt ist.

6. Verbindung nach einem der Ansprüche 1 bis 5, worin das ungesättigte 5- bis 10-gliedrige Ringsystem in R² gegebenenfalls durch mindestens einen unter Halogen, Nitro, C₂-C₆-Alkenyl, C₁-C₆-Alkoxy, C₁-C₆-Alkoxycarbonyl, C₁-C₆-Alkylcarbonyl, Phenylcarbonyl, C₁-C₆-Alkyl, Isoxazolyl, Pyrazolyl, -NR¹²R¹³, -C(O)NR¹⁴R¹⁵, -NHC(O)NR¹⁶R¹⁷ und -NHC(O)R²³ ausgewählten Substituenten substituiert ist.

7. Verbindung nach Anspruch 1, ausgewählt unter:
N-[2-({4-(4-Fluorphenyl)-4-oxobutyl]-2-morpholinyl}methoxy)phenyl]acetamid,
N-[2-({3-(4-Fluorphenyl)-3-oxopropyl]-2-morpholinyl}methoxy)phenyl]acetamid,
N-[2-({1-[4-(4-Fluorphenyl)-4-oxobutyl]-3-piperidinyl}methoxy)phenyl]acetamid,
N-[2-({4-(4-Fluorphenyl)-4-oxobutyl]-2-morpholinyl}methoxy)phenyl]acetamid,
1-(4-Fluorphenyl)-4-{2-[3-methoxyphenoxy)methyl]-4-morpholinyl}-1-butanon,
N-[2-({(3R)-1-[4-(4-Fluorphenyl)-4-oxobutyl]-3-piperidinyl}methoxy)phenyl]acetamid,
N-[2-({(3S)-1-[4-(4-Fluorphenyl)-4-oxobutyl]-3-piperidinyl}methoxy)phenyl]acetamid,
N-[2-({1-[4-(4-Fluorphenyl)-4-oxobutyl]-3-piperidinyl}methoxy)phenyl]benzamid,
4-{3-[(2-Acetylphenoxy)methyl]-1-piperidinyl}-1-(4-fluorphenyl)-1-butanon,
4-{3-[(3-Benzoylphenoxy)methyl]-1-piperidinyl}-1-(4-fluorphenyl)-1-butanon,
4-{3-[(1,3-Benzothiazol-2-yloxy)methyl]-1-piperidinyl}-1-(4-fluorphenyl)-1-butanon,
1-(4-Fluorphenyl)-4-{3-[(8-chinolinyloxy)methyl]-1-piperidinyl}-1-butanon,
1-(4-Fluorphenyl)-4-(3-{[(2-methyl-8-chinolinyl)-oxy]methyl}-1-piperidinyl)-1-butanon,
1-(4-Fluorphenyl)-4-{3-[(4-chinazolinyloxy)-methyl]-1-piperidinyl}-1-butanon,
1-(4-Fluorphenyl)-4-{3-[(1-naphthyloxy)methyl]-1-piperidinyl}-1-butanon,
1-(4-Fluorpheny)-4-(3-{[2-(5-isoxazolyl)phenoxy]-methyl}-1-piperidinyl)-1-butanon,
4-(3-{[4-Chlor-2-(1H-pyrazol-5-yl)phenoxy]methyl}-1-piperidinyl)-1-(4-fluorphenyl)-1-butanon,
N-Cyclopropyl-2-({1-[4-(4-fluorphenyl)-4-oxo-butyl]-3-piperidinyl}methoxy)benzamid,
2-({1-[4-(4-Fluorphenyl)-4-oxobutyl]-3-piperidinyl}methoxy)benzoesäuremethylester,
2-({1-[4-(4-Fluorphenyl)-4-oxobutyl]-3-piperidinyl}methoxy)-N-methylbenzamid,
1-(4-Fluorphenyl)-4-[3-({2-[(E)-1-propenyl]-phenoxy}methyl)-1-piperidinyl]-1-butanon,
4-{3-[3,5-Difluorphenoxy)methyl]-1-piperidinyl}-1-(4-fluorphenyl)-1-butanon,
4-{3-[(4-Chlor-2-isopropyl-5-methylphenoxy)-methyl]-1-piperidinyl}-1-(4-fluorphenyl)-1-butanon,
4-{3-[3,4-Dinitrophenoxy)methyl]-1-piperidinyl}-1-(4-fluorphenyl)-1-butanon,
1-(4-Fluorphenyl)-4-{3-[(2-isopropoxyphenoxy)-methyl]-1-piperidinyl}-1-butanon,
1-(4-Fluorphenyl)-4-{3-[(2-propionylphenoxy)-methyl]-1-piperidinyl}-1-butanon,
4-{3-[(2-Butyrylphenoxy)methyl]-1-piperidinyl}-1-(4-fluorphenyl)-1-butanon,
1-(4-Fluorphenyl)-4-(3-{[(3-methyl-1,2-benzisoxazol-4-yl)oxy]methyl}-1-piperidinyl)-1-butanon,
N-[2-({1-[4-(4-Fluorphenyl)-4-oxobutyl]-3-piperidinyl}methoxy)phenyl]harnstoff,
5-Chlor-2-({1-[4-(4-fluorphenyl)-4-oxobutyl]-3-piperidinyl}methoxy)benzamid,
N-Ethyl-N'-[2-({1-[4-(4-fluorphenyl)-4-oxobutyl]-3-piperidinyl}methoxy)phenyl]harnstoff,
N'-[2-({1-[4-(4-Fluorphenyl)-4-oxobutyl]-3-piperidinyl}methoxy)phenyl]-N,N-dimethylharnstoff,
4-(3-{[2-(Benzylamino)phenoxy]methyl}-1-piperidinyl)-1-(4-fluorphenyl)-1-butanon,
1-(4-Fluorphenyl)-4-(3-{[(2-methyl-1,3-benzoxazol-4-yl)oxy]methyl}-1-piperidinyl)-1-butanon,
und pharmazeutisch annehmbare Salze und Solvate davon.

8. Verfahren zur Herstellung einer Verbindung der Formel (I) gemäß Anspruch 1, bei dem man eine Verbindung der Formel 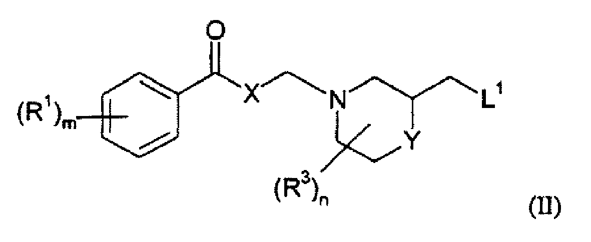 worin L¹ für eine Abgangsgruppe steht und m, n, X, Y, R¹ und R³ die unter Formel (I) angegebene Bedeutung besitzen, mit einer Verbindung der Formel
R²-OH (II)
worin R² die unter Formel (I) angegebene Bedeutung besitzt, umsetzt;
und gegebenenfalls danach ein pharmazeutisch annehmbares Salz oder Solvat der erhaltenen Verbindung der Formel (I) bildet.

9. Pharmazeutische Zusammensetzung, die eine Verbindung der Formel (I) oder ein pharmazeutisch annehmbares Salz oder Solvat davon nach einem der Ansprüche 1 bis 7 zusammen mit einem pharmazeutisch annehmbaren Hilfsstoff, Verdünnungsmittel oder Träger enthält.

10. Verfahren zur Herstellung einer pharmazeutischen Zusammensetzung nach Anspruch 9, bei dem man eine Verbindung der Formel (I) oder ein pharmazeutisch annehmbares Salz oder Solvat davon nach einem der Ansprüche 1 bis 7 mit einem pharmazeutisch annehmbaren Hilfsstoff, Verdünnungsmittel oder Träger vermischt.

11. Verbindung der Formel (I) oder ein pharmazeutisch annehmbares Salz oder Solvat davon nach einem der Ansprüche 1 bis 7 zur Verwendung bei der Therapie.

12. Verwendung einer Verbindung der Formel (I) oder eines pharmazeutisch annehmbaren Salzes oder Solvats davon nach einem der Ansprüche 1 bis 7 bei der Herstellung eines Arzneimittels zur Behandlung von Erkrankungen oder Beschwerden des Menschen, bei denen die Modulierung der Chemokinrezeptoraktivität von Vorteil ist.

13. Verwendung einer Verbindung der Formel (I) oder eines pharmazeutisch annehmbaren Salzes oder Solvats davon nach einem der Ansprüche 1 bis 7 bei der Herstellung eines Arzneimittels zur Verwendung bei der Behandlung von rheumatoider Arthritis.

14. Verwendung einer Verbindung der Formel (I) oder eines pharmazeutisch annehmbaren Salzes oder Solvats davon nach einem der Ansprüche 1 bis 7 bei der Herstellung eines Arzneimittels zur Verwendung bei der Behandlung von chronisch obstruktiver Lungenerkrankung.

15. Verwendung einer Verbindung der Formel (I) oder eines pharmazeutisch annehmbaren Salzes oder Solvats davon nach einem der Ansprüche 1 bis 7 bei der Herstellung eines Arzneimittels zur Verwendung bei der Behandlung von Asthma.

16. Verwendung einer Verbindung der Formel (I) oder eines pharmazeutisch annehmbaren Salzes oder Solvats davon nach einem der Ansprüche 1 bis 7 bei der Herstellung eines Arzneimittels zur Verwendung bei der Behandlung von multipler Sklerose.

17. Verwendung einer Verbindung der Formel (I) oder eines pharmazeutisch annehmbaren Salzes oder Solvats davon nach einem der Ansprüche 1 bis 7 bei der Herstellung eines Arzneimittels zur Verwendung bei der Behandlung einer entzündlichen Erkrankung.

18. Verwendung einer Verbindung der Formel (I) oder eines pharmazeutisch annehmbaren Salzes oder Solvats davon nach einem der Ansprüche 1 bis 7 bei der Herstellung eines Arzneimittels zur Verwendung bei der Behandlung einer Atemwegserkrankung.

## Revendications

1. Composé de la formule 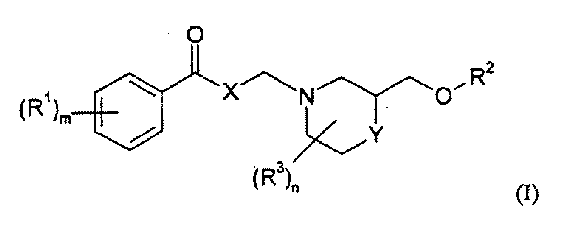 dans laquelle
m vaut 0, 1, 2 ou 3 ;
chaque R¹ représente indépendamment un groupe halogène, cyano, nitro, carboxyle, hydroxyle, cycloalkyle de C₃ à C₆, alcoxy de C₁ à C₆, alcoxycarbonyle de C₁ à C₆, haloalkyle de C₁ à C₆, haloalcoxy de C₁ à C₆, -NR⁶R⁷, cycloalkylamino de C₃ à C₆, alkylthio de C₁ à C₆, alkylcarbonyle de C₁ à C₆, alkylcarbonylamino de C₁ à C₆, sulfonamide, alkylsulfonyle de C₁ à C₆, -C(O)NR⁸R⁹, - NR¹⁰C(O)-(NH)ₚR¹¹, phényle ou alkyle de C₁ à C₆, éventuellement substitué par au moins un substituant choisi parmi un groupe carboxyle et un groupe alcoxycarbonyle de C₁ à C₆ ;
X représente une liaison ou un groupe CH₂ ou un groupe (CH₂)₂ ;
Y représente un atome d'oxygène ou un groupe CH₂ ;
R² représente un système cyclique non saturé de cinq à dix chaînons qui peut comprendre au moins un hétéroatome cyclique choisi par les atomes de l'azote, de l'oxygène et du soufre, le système cyclique étant éventuellement substitué par au moins un substituant choisi parmi les groupes halogène, cyano, nitro, carboxyle, hydroxyle, alcényle de C₂ à C₆, cycloalkyle de C₃ à C₅, alcoxy de C₁ à C₆, alcoxycarbonyle de C₁ à C₆, haloalkyle de C₁ à C₆, haloalcoxy de C₁ à C₆, - NR¹²R¹³, cycloalkylamino de C₃ à C₆, alkylthio de C₁ à C₆, alkylcarbonyle de C₁ à C₆, phénylcarbonyle, alkylcarbonylamino de C₁ à C₆, sulfonamide, alkylsulfonyle de C₁ à C₆, -C(O)NR¹⁴R¹⁵, alcoxycarbonyle de C₁ à C₆-alkyle de C₁ à C₆, phényle, isoxazolyle, pyrazolyle, -NHSO₂CH₃, -NHC(O)NR¹⁶R¹⁷, -OC(O)NR¹⁸R¹⁹, - OCH₂C(O)NR²⁰R²¹, -NHC(O)OR²², -NHC(O)R²³ et alkyle de C₁ à C₆, lui-même éventuellement substitué par au moins un substituant choisi parmi le groupe carboxyle et le groupe alcoxycarbonyle de C₁ à C₆;
n vaut 0, 1 ou 2 ;
chaque R³ représente indépendamment un groupe alkyle de C₁ à C₆, un groupe alcoxycarbonyle de C₁ à C₆, un groupe -CH₂OH ou un groupe carboxyle ;
R⁶ et R⁷ représentent chacun indépendamment un atome d'hydrogène ou un groupe alkyle de C₁ à C₆, ou R⁶ et R⁷ ensemble avec l'atome d'azote auquel ils sont rattachés forment un hétérocycle saturé de quatre à sept chaînons ;
R⁸ et R⁹ représentent chacun indépendamment un atome d'hydrogène ou un groupe alkyle de C₁ à C₆ éventuellement substitué par au moins un groupe alcoxycarbonyle de C₁ à C₆ ;
p vaut 0 ou 1 ;
R¹⁰ représente un atome d'hydrogène ou un groupe alkyle de C₁ à C₆ ;
R¹¹ représente un atome d'hydrogène ou un groupe alkyle de C₁ à C₆ éventuellement substitué par au moins un substituant choisi parmi les groupes carboxyle, alcoxy de C₁ à C₆ ou alcoxycarbonyle de C₁ à C₆ ;
R¹² et R¹³ représentent chacun indépendamment un atome d'hydrogène ou un groupe alkyle de C₁ à C₆ éventuellement substitué par au moins un substituant choisi parmi les groupes phényle, carboxyle et alcoxycarbonyle de C₁ à C₆, ou R¹² et R¹³ ensemble avec l'atome d'azote auquel ils sont rattachés forment un hétérocycle saturé de quatre à sept chaînons ;
R¹⁴ et R¹⁵ représentent chacun indépendamment un atome d'hydrogène, un groupe cycloalkyle de C₃ à C₆ ou un groupe alkyle de C₁ à C₆ éventuellement substitué par au moins un substituant choisi parmi les groupes carboxyle et alcoxycarbonyle de C₁ à C₆, ou R¹⁴ et R¹⁵ ensemble avec l'atome d'azote auquel ils sont rattachés forment un hétérocycle saturé de quatre à sept chaînons ;
R¹⁶ et R¹⁷ représentent chacun indépendamment un atome d'hydrogène ou un groupe alkyle de C₁ à C₆ éventuellement substitué par au moins un substituant choisi parmi les groupes carboxyle et alcoxycarbonyle de C₁ à C₆, ou R¹⁶ et R¹⁷ ensemble avec l'atome d'azote auquel ils sont rattachés forment un hétérocycle saturé de quatre à sept chaînons ;
R¹⁸ et R¹⁹ représentent chacun indépendamment un atome d'hydrogène ou un groupe alkyle de C₁ à C₆ éventuellement substitué par au moins un substituant choisi parmi les groupes carboxyle et alcoxycarbonyle de C₁ à C₆, ou R¹⁸ et R¹⁹ ensemble avec l'atome d'azote auquel ils sont rattachés forment un hétérocycle saturé de quatre à sept chaînons ;
R²⁰ et R²¹ représentent chacun indépendamment un atome d'hydrogène ou un groupe alkyle de C₁ à C₆ éventuellement substitué par au moins un substituant choisi parmi les groupes carboxyle et alcoxycarbonyle de C₁ à C₆, ou R²⁰ et R²¹ ensemble avec l'atome d'azote auquel ils sont rattachés forment un hétérocycle saturé de quatre à sept chaînons ;
R²² représente un atome d'hydrogène ou un groupe alkyle de C₁ à C₆ éventuellement substitué par au moins un substituant choisi parmi les groupes carboxyle et alcoxycarbonyle de C₁ à C₆ ;
R²³ représente un groupe alkyle de C₁ à C₆, un groupe alcényle de C₂ à C₆, un groupe cycloalkyle de C₃ à C₆, un groupe adamantyle, un groupe cycloalcényle de C₅ à C₆, un groupe phényle ou un système hétérocyclique saturé ou non saturé de cinq à dix chaînons, qui comprend au moins un hétéroatome cyclique choisi parmi les atomes d'azote, d'oxygène et de soufre, chaque groupe étant éventuellement substitué par au moins un substituant choisi parmi les groupes nitro, hydroxyle, oxo, halogène, carboxyle, alkyle de C₁ à C₆, alcoxy de C₁ à C₆, alkylthio de C₁ à C₆, alkylcarbonyle de C₁ à C₆, alcoxycarbonyle de C₁ à C₆, phényle et -NHC(O)-R²⁴ ; et
R²⁴ représente un groupe alkyle de C₁ à C₆, un groupe amino (-NH₂) ou un groupe phényle ;
ou un sel ou un solvate pharmaceutiquement acceptable de ceux-ci.

2. Composé selon la revendication 1, dans lequel chaque R¹ représente indépendamment un groupe halogène, un groupe cyano, un groupe nitro, un groupe alcoxy de C₁ à C₆, un groupe alkylcarbonyle de C₁ à C₆ ou un groupe alkylcarbonylamino de C₁ à C₆.

3. Composé selon la revendication 1 ou la revendication 2, dans lequel X représente un groupe CH₂ ou un groupe (CH₂)₂.

4. Composé selon l'une quelconque des revendications 1 à 3, dans lequel Y représente un groupe CH₂.

5. Composé selon l'une quelconque des revendications 1 à 4, dans lequel le système cyclique non saturé de cinq à dix chaînons dans R² est choisi parmi les groupes phényle, naphtyle, 1,3-benzodioxolyle, pyrazolyle, thiényle, oxazolyle, imidazolyle, pyridinyle, pyridopyrrolyle, benzimidazolyle, indazolyle, benzothiazolyle, quinolinyle, isoquinolinyle, quinazolinyle, quinoxalinyle, benzisoxazolyle, benzoxazolyle, thiazolyle et benzotriazolyle.

6. Composé selon l'une quelconque des revendications 1 à 5, dans lequel le système cyclique non saturé de cinq à dix chaînons dans R² est éventuellement substitué par au moins un substituant choisi parmi les groupes halogène, nitro, alcényle de C₂ à C₆, alcoxy de C₁ à C₆, alcoxycarbonyle de C₁ à C₆, alkylcarbonyle de C₁ à C₆, phénylcarbonyle, alkyle de C₁ à C₆, isoxazolyle, pyrazolyle, -NR¹²R¹³, -C(O)NR¹⁴R¹⁵, - NHC(O)NR¹⁶R¹⁷ et -NHC(O)R²³_{.}

7. Composé selon la revendication 1, qui est choisi parmi le groupe suivant :
N-[2-({4-(4-fluorophényl)-4-oxobutyl]-2-morpholinyl}méthoxy)phényl]acétamide,
N-[2-({3-(4-fluorophényl)-3-oxopropyl]-2-morpholinyl}méthoxy)phényl]acétamide,
N-[2-({1-[4-(4-fluorophényl)-4-oxobutyl)-3-pipéridinyl}méthoxy)phényl]acétamide,
N-[2-({4-(4-fluorophényl)-4-oxobutyl]-2-morpholinyl}méthoxy)phényl]acétamide,
1-(4-fluorophényl)-4-{2-[3-méthoxyphénoxy)méthyl]-4-morpholinyl}-1-butanone,
N-[2-({(3R)-1-[4-(4-fluorophényl)-4-oxobutyl]-3-pipéridinyl}méthoxy)phényl]acétamide,
N-[2-({(3S)-1-[4-(4-fluorophényl)-4-oxobutyl]-3-pipéridinyl}méthoxy)phényl]acétamide,
N-[2-({1-[4-(4-fluorophényl)-4-oxobutyl)-3-pipéridinyl}méthoxy)phényl]-benzamide,
4-{3-[(2-acétylphénoxy)méthyl]-1-pipéridinyl}-1-(4-fluorophényl)-1-butanone,
4-{3-[(3-benzoylphénoxy)méthyl]-1-pipéridinyl}-1-(4-fluorophényl)-1-butanone,
4-{3-[(1,3-benzothiazol-2-yloxy)méthyl]-1-pipéridinyl}-1-(4-fluorophényl)-1-butanone,
1-(4-fluorophényl)-4-{3-[(8-quionolinyloxy)méthyl]-1-pipéridinyl}-1-butanone,
1-(4-fluorophényl)-4-{3-{[(2-méthyl-8-quionolinyl)oxy]méthyl}-1-pipéridinyl)-1-butanone,
1-(4-fluorophényl)-4-{3-[(4-quionolinyloxy)méthyl]-1-pipéridinyl}-1-butanone,
1-(4-fluorophényl)-4-{3-[(1-naphtyloxy)méthyl]-1-pipéridinyl}-1-butanone,
1-(4-fluorophényl)-4-{3-{[2-(5-isoxazolyl)phénoxy]méthyl}-1-pipéridinyl)-1-butanone,
4-(3-{[4-chloro-2-(1H-pyrazol-5-yl)phénoxy]méthyl}-1-pipéridinyl)-1-(4-fluorophényl)-1-butanone,
N-cyclopropyl-2-({1-[4-(4-fluorophényl)-4-oxobutyl}-3-pipéridinyl}méthoxy)-benzamide,
2-({1-[4-(4-fluorophényl)-4-oxobutyl]-3-pipéridinyl}méthoxy)benzoate de méthyle,
2-({1-[4-(4-fluorophényl)-4-oxobutyl]-3-pipéridinyl}méthoxy)-N-méthylbenzamide,
1-(4-fluorophényl)-4-[3-({2-[(E)-1-propényl]phénoxy}méthyl)-1-pipéridinyl]-1-butanone,
4-{3-[3,5-difluorophénoxy)méthyl]-1-pipéridinyl}-1-(4-fluorophényl)-1-butanone,
4-[3[(4-chloro-2-isopropyl-5-méthylphénoxy)méthyl]-1-pipéridinyl}-1-(4-fluorophényl)-1-butanone,
4-{3-[3,4-dinitrophénoxy)méthyl]-1-pipéridinyl}-1-(4-fluorophényl)-1-butanone,
1-(4-fluorophényl)-4-{3-[(2-isopropoxyphénoxy)méthyl]-1-pipéridinyl}-1-butanone,
1-(4-fluorophényl)-4-{3-[(2-propionylphénoxy)méthyl]-1-pipéridinyl}-1-butanone,
4-[3[(2-butyrylphénoxy)méthyl]-1-pipéridinyl}-1-(4-fluorophényl)-1-butanone,
1-(4-fluorophényl)-4-{3-{[(3-méthyl-1,2-benzisoxazol-4-yl)-oxy]méthyl}-1-pipéridinyl}-1-butanone,
N-[2-({1-[4-(4-fluorophényl)-4-oxobutyl]-3-pipéridinyl}méthoxy)phényl]urée,
5-chloro-2-({1-[4-(4-fluorophényl)-4-oxobutyl]-3-pipéridinyl}méthoxy)benzamide,
N-éthyl-N'-[2-({1-[4-(4-fluorophényl)-4-oxobutyl]3-pipéridinyl}méthoxy)-phényl]urée,
N'-[2-({1-[4-(4-fluorophényl)-4-oxobutyl]-3-pipéridinyl}méthoxy)phényl]-N,N-diméthylurée,
4-(3-{[2-(benzylamino)phénoxy]méthyl}-1-pipéridinyl)-1-(4-fluorophényl)-1-butanone,
1-(4-fluorophényl)-4-(3-{[(2-méthyl-1,3-benzoxazol-4-yl)oxy]méthyl}-1-pipéridinyl)-1-butanone,
et des sels et des solvates pharmaceutiquement acceptables de l'un quelconque de ceux-ci.

8. Procédé pour la préparation d'un composé de la formule (I) selon la revendication 1, lequel comprend la réaction d'un composé de la formule 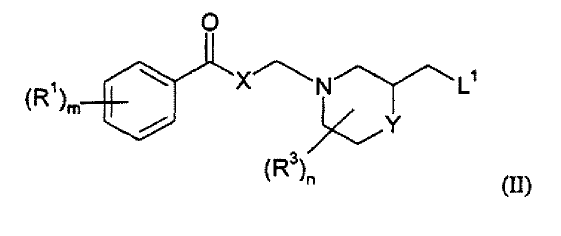 dans laquelle L¹ représente un groupe partant et m, n, X, Y, R¹ et R³ sont tels que définis dans la formule (I), avec un composé de la formule
R² - OH (II)
dans laquelle R² est tel que défini dans la formule (I) ;
et forme éventuellement par la suite un sel ou un solvate pharmaceutiquement acceptable du composé de la formule (I) obtenu.

9. Composition pharmaceutique comprenant un composé de la formule I, ou un sel ou un solvate pharmaceutiquement acceptable de celui-ci, selon l'une quelconque des revendications 1 à 7, en association avec un adjuvant, un diluant ou un vecteur pharmaceutiquement acceptable.

10. Procédé pour la préparation d'une composition pharmaceutique selon la revendication 9, lequel comprend le mélange d'un composé de la formule (I), ou d'un sel ou d'un solvate pharmaceutiquement acceptable de celui-ci, selon l'une quelconque des revendications 1 à 7, avec un adjuvant, un diluant ou un vecteur pharmaceutiquement acceptable.

11. Composé de la formule I, ou un sel ou un solvate pharmaceutiquement acceptable de celui-ci selon l'une quelconque des revendications 1 à 7, pour utilisation dans une thérapie.

12. Utilisation d'un composé de la formule (I), ou d'un sel ou d'un solvate pharmaceutiquement acceptable de celui-ci, selon l'une quelconque des revendications 1 à 7, dans la fabrication d'un médicament pour le traitement de maladies ou de troubles humains dans lesquels la modulation de l'activité du récepteur de chimiokine est bénéfique.

13. Utilisation d'un composé de la formule (I), ou d'un sel ou d'un solvate pharmaceutiquement acceptable de celui-ci, selon l'une quelconque des revendications 1 à 7, dans la fabrication d'un médicament pour utilisation dans le traitement de l'arthrite rhumatoïde.

14. Utilisation d'un composé de la formule (I), ou d'un sel ou d'un solvate pharmaceutiquement acceptable de celui-ci, selon l'une quelconque des revendications 1 à 7, dans la fabrication d'un médicament pour utilisation dans le traitement de la maladie pulmonaire obstructive chronique.

15. Utilisation d'un composé de la formule (I), ou d'un sel ou d'un solvate pharmaceutiquement acceptable de celui-ci, selon l'une quelconque des revendications 1 à 7 dans la fabrication d'un médicament pour utilisation dans le traitement de l'asthme.

16. Utilisation d'un composé de la formule (I), ou d'un sel ou d'un solvate pharmaceutiquement acceptable de celui-ci, selon l'une quelconque des revendications 1 à 7 dans la fabrication d'un médicament pour utilisation dans le traitement de la sclérose en plaques.

17. Utilisation d'un composé de la formule (I), ou d'un sel ou d'un solvate pharmaceutiquement acceptable de celui-ci, selon l'une quelconque des revendications 1 à 7, dans la fabrication d'un médicament pour utilisation dans le traitement d'une maladie inflammatoire.

18. Utilisation d'un composé de la formule (I), ou d'un sel ou d'un solvate pharmaceutiquement acceptable de celui-ci, selon l'une quelconque des revendications 1 à 7, dans la fabrication d'un médicament pour utilisation dans le traitement d'une maladie des voies respiratoires.
